(19) Europäisches Patentamt
European Patent Office
Office européen des brevets

(11) EP 2 850 210 B1

(12) **EUROPEAN PATENT SPECIFICATION**

(45) Date of publication and mention of the grant of the patent:
**12.09.2018 Bulletin 2018/37**

(21) Application number: **13732214.5**

(22) Date of filing: **17.05.2013**

(51) Int Cl.:
***C12Q 1/68*** *(2018.01)*

(86) International application number:
**PCT/GB2013/051278**

(87) International publication number:
**WO 2013/171504 (21.11.2013 Gazette 2013/47)**

(54) **METHODS TO ASSESS THE LIKELIHOOD OF DYSPLASIA OR ESOPHAGEAL ADENOCARCINOMA**

VERFAHREN ZUR BEURTEILUNG DER WAHRSCHEINLICHKEIT VON DYSPLASIEN ODER SPEISERÖHRENADENOKARZINOMEN

PROCÉDÉS D'ÉVALUATION DE PROBABILITÉ D'UNE DYSPLASIE OU D'UN ADÉNOCARCINOME OESOPHAGIEN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.05.2012 GB 201208874**

(43) Date of publication of application:
**25.03.2015 Bulletin 2015/13**

(73) Proprietor: **United Kingdom Research and Innovation**
**Swindon SN2 1FL (GB)**

(72) Inventors:
- **FITZGERALD, Rebecca**
  **Cambridge,**
  **Cambridgeshire CB2 2XZ (GB)**
- **ALVI, Muhammad**
  **Cambridge,**
  **Cambridgeshire CB2 2XZ (GB)**
- **LIU, Xinxue**
  **Cambridge,**
  **Cambridgeshire CB2 2XZ (GB)**

(74) Representative: **Script IP Limited**
**Turnpike House**
**18 Bridge Street**
**Frome Somerset BA11 1BB (GB)**

(56) References cited:
**WO-A2-2009/105533 US-A1- 2012 094 287**

- **ALVI MUHAMMAD A ET AL: "DNA Methylation is a Biomarker for Dysplasia and Early-Stage Neoplasia in Barrett's Esophagus: From Discovery to a Phase 4 Prospective Validation Study", GASTROENTEROLOGY, vol. 142, no. 5, Suppl. 1, 5 May 2012 (2012-05-05), page S443, XP008165040, & DIGESTIVE DISEASE WEEK (DDW); SAN DIEGO, CA, USA; MAY 19 -22, 2012**
- **KAZ A M ET AL: "DNA methylation profiling in Barrett's esophagus and esophageal adenocarcinoma reveals unique methylation signatures and molecular subclasses", EPIGENETICS 2011 LANDES BIOSCIENCE USA, vol. 6, no. 12, 2011, pages 1403-1412, XP002713856, ISSN: 1559-2294**
- **JIN Z ET AL: "197 A Multicenter, Double-Blinded Validation Study of Methylation Biomarkers for Progression Prediction in Barrett's Esophagus", GASTROENTEROLOGY, ELSEVIER, PHILADELPHIA, PA, vol. 136, no. 5, 1 May 2009 (2009-05-01), pages A-36, XP026110715, ISSN: 0016-5085, DOI: 10.1016/S0016-5085(09)60168-2 [retrieved on 2009-05-01]**
- **M. A. ALVI ET AL: "DNA Methylation as an Adjunct to Histopathology to Detect Prevalent, Inconspicuous Dysplasia and Early-Stage Neoplasia in Barrett's Esophagus", CLINICAL CANCER RESEARCH, vol. 19, no. 4, 15 February 2013 (2013-02-15), pages 878-888, XP055081565, ISSN: 1078-0432, DOI: 10.1158/1078-0432.CCR-12-2880**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### Background to the invention

Patients with Barrett's esophagus (BE) have a substantially increased risk of progression to esophageal adenocarcinoma (EAC) compared to the general population (RR: 11.3, 95% CI: 8.8-14.4)1. The incidence of EAC has increased 7-fold in the past 30 years (3.6 to 25.6 cases per million)2 and the prognosis is poor with a median survival of about 11 months due to late presentation3. Due to the improved survival in those diagnosed when the disease is confined to mucosa or sub-mucosal layers; patients with BE are recommended to undergo endoscopic surveillance for the early detection of cancer4, 5. The cost-effectiveness and risk:benefit ratio to the patient of endoscopy has been questioned time and again since the annual (per year) risk of progression is relatively low1, 6, 7; around 0.3% according to the recent estimates8. The intermediate dysplastic stages between BE and EAC are the most reliable marker of progression; however the histological presence of dysplasia is subjective due to known sampling bias during endoscopy along with a high inter and intra-observer variability9, 10. The wide variation in progression rates in patients categorized as having low grade dysplasia has been highlighted by two recent studies. In a Dutch study the incidence rate of high grade dysplasia (HGD) or EAC in individuals with confirmed low grade dysplasia was high at 13.4% (95% CI 3.5 - 23.2) per patient per annum 11; whereas in patients in a US study the progression rate of this group was similar to that of non-dysplastic patients which is a 16-fold difference12. In patients with high grade dysplasia, data from a randomized radiofrequency ablation (RFA) intervention trial suggest a rate of progression of 19% in the non-treatment arm13. Hence there is a high need for biomarkers that can accurately detect prevalent dysplasia in flat Barrett's mucosa and predict those patients most likely to progress to cancer.

Aberrant DNA methylation is shown to be a characteristic of cancer and these changes are known to occur early during transformation 14. It has already been shown in a number of studies that DNA methylation changes occur during progression from BE to EAC and that these alterations have the potential to be used as biomarkers15-20. These studies have mostly employed a candidate approach based on known methylation targets in other cancers. However high-throughput array based platforms are now available to identify DNA methylation changes and we have employed this approach to find candidate biomarkers in Barrett's carcinogenesis. Imprinted genes and the X-chromosome are both epigenetically controlled by DNA methylation21, but have never been examined specifically in the context of biomarkers for EAC.

Jin et al (2009 Cancer Research v.69, pages 4112 to 4115) disclose a multicentre, double-blinded validation study of methylation biomarkers for progression prediction in Barrett's esophagus. The authors studied eight BE progression prediction methylation biomarkers. The authors studied methylation in 145 non-progressors and 50 progressors from Barrett's esophagus to neoplasia. The study was a retrospective study - the study candidate genes assessed were p16, for methylation status *a posteriori*. The eight CDH13. The authors suggest that their eight marker panel is more objective and quantifiable and possesses higher predictive sensitivity and specificity than assessment of clinical features such as age. The authors assert that their eight marker panel accurately predicted approximately half of HGDs and EACs at high specificity levels.

Kaz et al (2011 Epigenetics v.6, pages 1403 to 1412) disclose that DNA methylation profiling in Barrett's esophagus and esophagal adenocarcinoma reveals unique methylation signatures and molecular subclasses. The authors report the finding of distinct global methylation that their signatures could methylation of specific genes. The authors claim that their signatures could discriminate between squamous, BE, HGD and EAC cells. The authors do not disclose any biomarkers. Indeed, the authors even concede this when they state "Additional validation of those CpG sites that distinguished BE from when they and EAC may lead to the discovery CpG sites biomarkers with potential clinical applications in the diagnosis and prognosis of BE and EAC". Thus, this report is focused on a description of CpG methylation profiles. In particular, the methylation status of 1505a description spread methylation profiles. In studied. No specific teachings of biomarkers or prognosis are provided in this document.

D1 - ALVI et al, 2012 (Gastroenterology, & Digestive Disease Week (DDW); SAN DIEGO, CA, USA; vol. 142, no. 5, Suppl. 1, page S443) discloses that DNA methylation is a biomarker for dysplasia and early-stage neoplasia in Barrett's esophagus. They disclosed an approach from discovery to a phase 4 prospective validation study.

D2 - WO2009/1005533 (UNIV JOHNS HOPKING [US], et al) discloses methodes for predicting esophageal adenocarcinoma (EAC). The study focuses on methylation levels of transcriptional promoter region of various combinations of cadherins such as cadherin 13, H-cadherin (heart), and other genes.

D3 - US2012094287 (MARKOWITZ SANFORD D [US], et al) discloses methods and compositions for detecting gastrointestinal and other cancers. In particular the disclosure focuses on methods and compositions for detecting and treating vimentin-associated neoplasia, including differential methylation of the vimentin nucleotide sequences.

D4 - KAZ et al 2011 (EPIGENETICS LANDES BIOSCIENCE USA, vol. 6, no. 12, ISSN 1559-2294, pages 1403 - 1412) discloses that DNA methylation profiling in Barrett's esophagus and esophageal adenocarcinoma reveals unique methylation signatures and molecular subclasses.

D5 - JIN et al 2009 (GASTROENTEROLOGY, ELSEVIER, PHILADELPHIA, PA, vol. 136, no. 5 pages A - 36) discloses a multicenter, double-blinded validation study of methylation biomarkers for progression prediction in Barrett's esophagus.
The present invention seeks to overcome problem(s) associated with the prior art.

## Summary of the Invention

The invention is defined by the appended claims.
The inventors addressed the key issue of endoscopic surveillance of Barrett's esophagus (BE) and suggest how DNA methylation alterations can improve detection of high grade dysplasia and early cancer in flat Barrett's mucosa alongside histopathology.
To do this we have conducted a high-throughput array based methylation scan and utilised rigorous statistical methods (signal-to-noise ration and two sided Wilcoxon tests) to rank differentially methylated genes on the Illumina Infinium platform. In addition, we have specifically looked at imprinted and X-chromosome genes for any changes in methylation occurring during the course of cancer development since these genes may be ideal biomarkers as physiological inactivation of one allelic copy has already occured due to imprinting and via X-inactivation in females. Once we had identified candidate genes as biomarkers we then performed robust validation using pyrosequencing on the same samples as well as in a large independent set of retrospectively collected samples. This led to the identification of a four gene methylation panel which could distinguish between patients with non-dysplasctic compared with dysplastic Barrett's and early carcinoma. Finaly, we took this forward to a prospective, multicenter study and demonstrated that this panel does have clinical utility. Hence, we have gone from discovery all the way through to prospective evaluation of our panel.
Endoscopic surveillance of Barrett's esophagus (BE) is problematic because dysplasia and early-stage neoplasia are frequently invisible and likely to be missed due to sampling bias. Molecular abnormalities may be more diffuse than dysplasia. The aim was therefore to test whether DNA methylation; especially on imprinted and X-chromosome genes; is able to detect dysplasia/early-stage neoplasia.
We describe a surprisingly robust panel of methylation markers which correlate with useful clinical indications. A key further advantage of the invention is the use of the field effect whereby the invention can help overcome sampling bias since the informative markers taught occur in the Barrett's lesion and not solely in the dysplastic/EAC region (if present). The invention is based on these surprising findings.
Thus in one aspect the invention provides an *in vitro* method for aiding assessment of the likelihood of dysplasia or esophagal adenocarcinoma being present in subject, wherein the sample is from a subject having Barrett's Esophagus, the method comprising

(a) providing an esophagal sample from said subject
(b) determining the methylation status of

(i) SLC22A 18,
(ii) PIGR,
(iii) GJA 12 and
(iv) RIN2

in said sample;
comparing the methylation values of (b) to a reference standard, wherein said reference standard is from a subject having Barrett's esophagus, but not having dysplasia or esophagal adenocarcinoma;
wherein if 2 or more of said genes are methylated at a level higher than the reference standard then an increased likelihood of presence of dysplasia or esophagal adenocarcinoma is determined.
In one embodiment suitably step (a) comprises extracting nucleic acid such as DNA from said sample.
In one embodiment suitably step (b) comprises contacting said sample with a primer and determining methylation. Determining methylation may be carried out for example by MSP or pyrosequencing.
Suitably the method further comprises determining the methylation status of (v) TCEAL7.
Suitably if said subject is male, the method further comprises determining the methylation status of (vi) RGN.
Suitably the dysplasia is high grade dysplasia (HGD).
In another aspect, the invention relates to a method of assessing the risk for a particular subject comprising performing the method as described above, wherein if 0 or 1 of said genes are methylated then low risk is determined, and if 2 of said genes are methylated then intermediate risk is determined, if 3 or more of said genes are methylated then high risk is determined.
Depending on the outcome of the methods of the invention, alternate treatments may be offered to the subject.
For example, when a subject is identified as low risk according to the present invention, they may be prescribed surveillance at a longer time interval, for example three to five years.

When a subject is classified as intermediate risk according to the present invention, they may be prescribed a more frequent follow-up (a more frequent surveillance). For example, they may be prescribed a surveillance at a shortened interval such as one to two years.

When a subject is classified as high risk according to the present invention, they may be prescribed an intervention. For example, the subject may be prescribed a radio frequency ablation. This procedure is far more minor and less invasive than oesophagectomy. Therefore, the invention enables a more minor and less invasive treatment to be dispensed to patients who present in the high risk cactegory according to the invention.

For comparison, the current UK guidelines are that if a patient presents with a Barrett's esophagus segment of 3 cm or longer, they would be prescribed surveillance at approximately two to three year time intervals. Thus, it can be appreciated that the invention provides savings in surveillance costs by extending the time interval between surveillance for low risk patients, and also improves outcomes by allowing intervention at an earlier stage for high risk patients.

Suitably methylation status is determined by pyrosequencing.

Suitably said pyrosequencing is carried out using one or more sequencing primers selected from supplementary Table 5.

Suitably the methylation status is scored by determining the percentage methylation of each of said genes and comparing the values to the following methylation cut off percentages:

| Gene | Methylation cut-off (%) |
|---|---|
| GJA12 | 51.74000 |
| SLC22A18 | 49.25000 |
| PIGR | 64.755000 |
| RIN2 | 37.85500 |
| *RGN* (males only) | 18.645000 |
| TCEAL7 | 58.54000 |

wherein a value for a gene which exceeds the methylation cut off percentage for said gene is scored as 'methylated'.

Also described is an apparatus or system which is

(a) configured to analyse an esophagal sample from a subject, wherein said analysis comprises
(b) determining the methylation status of

(i) *SLC22A18*,
(ii) *PIGR*,
(iii) *GJA12* and
(iv) *RIN2*

in said sample,
said apparatus or system comprising an output module,
wherein if 2 or more of said genes are methylated then an increased likelihood of presence of dysplasia or esophagal methylated is determined. Suitably the analysis further comprises determining the methylation status of (v) TCEAL7. Suitably if said subject is male, the analysis further comprises determining the methylation status of (vi) RGN.

Suitably said sample comprises frozen biopsy material.

Also described is a method for aiding assessment of the likelihood of dysplasia or esophagal adenocarcinoma being present in a subject, the method comprising

(a) providing an esophagal sample from said subject
(b) determining the methylation

(i) *SLC22A18*,
(ii) *PIGR*,
(iii) *GJA12* and
(iv) *RIN2*

in said sample;
comparing the methylation values of (b) to a reference standard,
wherein if 2 or more of said genes are methylated at a level higher than the reference standard then an increased likelihood of presence of dysplasia or esophagal adenocarcinoma is determined.

Suitably said reference standard is from a subject having Barrett's esophagus, but not having dysplasia or esophagal adenocarcinoma.
Suitably said reference standard is from a Barrett's esophagus segment, but not having dysplasia or esophagal adenocarcinoma.
Suitably said reference standard comprises columar epithelium such as Barrett's esophagus or duodenum.

Suitably the method further comprises determining the methylation status of (v) TCEAL7. Suitably if said subject is male, the method further comprises determining the methylation status of (vi) RGN.

Also described is a computer program product operable, when executed on a computer, to perform the method steps as described above.

## Detailed Description of the Invention

We teach that DNA methylation can detect inconspicuous dysplasia and early-stage neoplasia in Barrett's esophagus, ie. that DNA methylation detects dysplasia/cancer.

Methylation changes in particular genes may be ideal biomarkers since physiological inactivation of one allelic copy may already have occurred due to imprinting and via X-inactivation in females.

We performed DNA methylation screening of BE and EAC samples using arrays to determine candidate biomarkers. We analyzed imprinted and X-chromosome genes separately and purposefully separated males from females to allow meaningful conclusions to be drawn. We performed robust internal and external validation using pyrosequencing which is the current gold standard in DNA methylation analysis and from this determined a panel of biomarkers to discriminate between dysplastic and non-dysplastic BE. Finally we validated the biomarker panel in a prospective cohort with real-time analysis to stratify BE patients into low, intermediate and high risk groups based on their risk of having prevalent dysplasia/EAC.

This study has identified widespread changes in DNA methylation which distinguish between BE and EAC. Use of an array based strategy has enabled us to identify novel genes previously unknown to play a role in this disease. We hypothesized that methylation of imprinted and X-chromosome genes might provide candidate biomarkers since one copy is already inactivated. The analysis demonstrated almost 70% imprinted genes had altered methylation status in EAC and one of these, *SLC 22A18,* was in the final stratification panel. Robust internal and external validation using pyrosequencing allowed us to select a four gene panel with an excellent receiver operating characteristic to distinguish between non-dysplastic BE and dysplastic BE/EAC samples (AUC=0.988). This panel enabled us to stratify patients into three (low, intermediate and high) risk groups based on the number of methylated genes identified from analysis of a limited number of biopsies by virtue of the field effect.

A number of previous studies have looked at DNA methylation changes in Barrett's carcinogenesis. However none of the genes such as *p16, APC*[24] and *MGMT*[19] and a previously identified eight gene panel[25] were shown in this current study to be differentially methylated in EAC vs. BE. One reason for this might be that most biomarker studies have used a candidate, rather than an array based approach, and compared the BE associated disease states (dysplasia and EAC) to the normal squamous epithelium of the esophagus whereas we have compared dysplasia/EAC to BE in our study[17, 26, 27]. Metaplastic BE resembles intestinal epithelium rather than the squamous esophagus; and there is the possibility that the differences in DNA methylation observed between the normal squamous esophageal epithelium and BE/dysplsia/EAC might purely reflect differences in tissue morphology rather than playing any role in carcinogenesis. For this reason we included two duodenum samples as control in our array based methylation scan. If the methylation level of a gene was similar in both BE and duodenum; it was deemed that gene was involved in the maintenance of the columnar intestinal type epithelium rather than in the development of cancer. There were also methodological differences in the assays used; previous studies have employed methylation specific PCR (MSP) whereas here we used pyrosequencing which is a more quantitative method that has gained widespread acceptance[28].

More hypermethylation was seen in cancer compared to hypomethylation (Table 1), in keeping with the fact that promoter hypermethylation is a well-established phenomenon in cancer. We also observed greater methylation changes to occur within known CpG islands. However in a recent publication comparing the normal squamous mucosa with Barrett's mucosa in 3 patients, methylation changes were reported to occur more frequently outside of CpG islands[22]. It should however be noted that the majority of probes on the Illumina Infinium platform are positioned around promoter sites and 60% of human genes are associated with promoters spanning CpG islands. The recent availability of comprehensive genome wide coverage of methylation changes will enable further light to be shed on this.

For imprinted genes, as mentioned above almost 70% of genes showed statistically significant changes in methylation in EAC vs. BE (Wilcoxon $P<0.05$) (Table 1). Disruption of genomic imprinting is a well-established phenomenon in cancer. One imprinted gene, *SLC 22A18,* met the criteria for validation. This gene is located in the 11p15.5 cluster which is an important tumor-suppressor gene region. Mutations, deletions and LOH of this gene have all been reported in different cancers highlighting its importance in tumorigenesis[30]. Gain of imprinting of *SLC22A18* has been documented in other cancers such as breast[31] and hepatocarcinomas[32] but we have shown for the first time that this can have a biomarker potential.

We looked at X-chromosome genes not only because DNA methylation plays a major role in X-inactivation in females but also because BE is more common in males who only have one copy of the X-chromosome and thus would theoretically only require one hit for the loss of the only functional allele. We were able to identify *RGN,* a putative tumor-suppressor gene[33, 34] that shows a successive increase in DNA methylation in the Barrett's associated metaplasia-dysplasia-adenocarcinoma sequence in males but not in females (Supplementary Figure 2).

Our findings have potential clinical applications. For the detection of dysplasia a four quadrant biopsy sampling technique is employed since dysplastic lesions can be focally distributed within the Barrett's segment without any endoscopically visible lesion. Furthermore, there is substantial intra-observer disagreement among pathologists in differentiating between low and high grade dysplasia[9, 10, 35]. In the prospective study we observed using our four gene methylation panel that DNA methylation is able to detect dysplasia/early-stage neoplasia in endoscopic biopsies even when the biopsy itself does not contain any visible dysplasia/early-stage neoplasia. This suggests that there is a field effect of methylation alterations in keeping with other research in the area of colon cancer[36, 37]. The clonality of BE and evolving dysplastic lesions is still not clearly understood[38, 39] but there do appear to be widespread molecular genetic changes prior to the emergence of phenotypical alteration visible by histopathology criteria[40]. Our methylation panel therefore has the potential to flag patients which do not show any visible signs of dysplasia/early-stage neoplasia but might still be at a high risk of progression. This needs validation in cohorts not skewed by referral bias in tertiary referral centers and is a promising area for further study.

**Field Effect**

It is an advantage of the invention that the particular biomarkers taught herein show the field effect. This means that any cells sampled within the Barrett's segment will show methylation according to the present invention if dysplasia or EAC is likely to be present somewhere within the Barrett's segment. Clinical practice is that the Barrett's segment is sampled at a number of places within the lesion. However, an area of dysplasia or EAC is typically smaller than the entire Barrett's lesion. Therefore, whether or not HGD/EAC is detected from a particular biopsy is largely affected by chance. If one or more of the samples taken from the Barrett's segment happens to be within the dysplasia/EAC, then positive results can be expected. However, due to sampling bias and/or laws of probability, it is quite possible to sample a Barrett's segment at a number of points, and yet none of those points happens to lie within a dysplastic or EAC area. In this situation, the patient would be returned a negative result. This would clearly be undesirable and potentially life-threatening for that patient. However, advantageously, according to the present invention, the markers which are assayed as indicative of risk of dysplasia/EAC are found throughout the Barrett's segment (not just in the dysplasia/EAC patch (es)). Therefore, by using the present invention the problem of "missing" the dysplasia/EAC due to sampling error is advantageously reduced or eliminated.

Another problem which can arise from sampling errors is a so-called "oscillating diagnosis". This refers to a situation where a first biopsy from a patient shows a negative result, a later biopsy shows a positive result, a still later biopsy shows a negative result and so on. This phenomenon arises due to probabilistic factors as outlined above, when the lesion such as dysplasia/EAC is smaller than the Barrett's segment being biopsied. It is typically only possible to see the dysplasia when it is in an advanced state and presents as a nodule or ulcer. More typically, the Barrett's segment is "flat", which means that no lumps/nodules or ulcers/holes are visible in the Barrett's segment. This means that when the endoscopist is collecting the biopsies, there is no way of focusing those biopsies on the possibly dysplastic lesion to be examined. The endoscopist then simply tries to collect samples across the whole surface of the Barrett's esophagus segment. However, as explained above, any such random sampling is prone to chance effects, and so in a certain proportion of cases a lesion may actually be present but will not be detected due to none of the samples having been taken from within the (invisible) dysplasia/EAC region. This presents problems for the physician when it looks like the dysplasia/EAC can be appearing or disappearing over time, which is of course extremely unlikely or impossible. However skilled the endoscopist is, marking or reproducing the sampling is extremely difficult. The only practical measure is using a graduated endoscope when the location of the samples is typically noted by distance from the incisors. It is problematic that this is a rather rough and unreliable estimate. For practical reasons, such as the markings on the endoscope being only every 10 cm, the distance measurement is typically only accurate to +/- 1-2cm. Moreover, in the case of repeat surveillance events, it is important to note that the taking of biopsies leaves no scarring or visible marker on the surface. Therefore, even if a patient is presenting for a repeat biopsy, the endoscopist has no opportunity to sample the same or different areas as were sampled in previous biopsies, since there is no marking or scarring visible from the earlier events. By using the present invention, these difficulties and drawbacks are advantageously overcome.

It should be noted that the invention is not concerned specifically with diagnosis. The invention is concerned with prediction and/or assessment of risk; in particular, the invention is useful in prediction of probability or risk of harbouring a lesion such as HGD or EAC. In particular the invention is useful in prediction of probability or risk of the subject harbouring EAC. In one embodiment dysplasia itself may be considered a risk factor for developing EAC. In one embodiment risk of EAC is the most important aspect to assess. The invention is suitably useful to aid in decisions about how a subject or patient should be treated or managed. The invention is most useful in ascribing a risk category to said subject or patient as described above.

### Definitions

The term 'comprises' (comprise, comprising) should be understood to have its normal meaning in the art, i.e. that the stated feature or group of features is included, but that the term does not exclude any other stated feature or group of features from also being present.

BE - Barrett's esophagus; BED - Barrett's esophagus with dysplasia; EAC - Esophageal adenocarcinoma; HGD - High grade dysplasia; LGD - Low grade dysplasia.

### Reference Sequences

Supplementary Table 5 provides details of the sequences of the genes of interest in the invention. Also provided are the addresses of the methylation regions of interest.

Suitably the reference sequences are as defined in the following table:

| Gene name (GenBank accession number) | CpG co ordinates | mRNA/Coding Sequence* |
|---|---|---|
| SLC22A18 (NM_ 183233.1) | 2877752 (+strand) | 1 gggggtacca gctccttact gccctgcaga caagcgtgcc gtgcgtgctt gtggccaagg<br>61 gaaggaagag ctggttgatc cacagatagc tccttcctcc ccgcccettc ctttttgttt<br>121 ggaggtccca ggatctgtgt tcacagacat ctgggggaag aaaaggagca ggaaactacc<br>181 ccgcacagag ttaagcagga aacaacaaca acatcatgca aaaaccctgc aaagaaaacg<br>241 aaggaaagcc aaagtgcagc gtgccaaaga gggaggaaaa acgcccgtat ggagaatttg<br>301 aacgccagca aacagaaggg aattttagac agaggctgct tcagtctctc gaagaattta<br>361 aagaggacat agactatagg cattttaaag atgaagaaat gacaagggag ggagatgaga<br>421 tggaaaggtg tttggaagag ataaggggtc tgagaaagaa atttagggct ctgcattcta<br>481 accataggca ttctcgggac cgtccttatc ccatttaatt aatttctctg acaattcaat<br>541 tattttctgt tattaatgtt gccactgctt tctgtttgtc tgcactttct tgataaatat<br>601 ttgctatcgt tttactccag tcattcgatg ttgctgagat ttacatatga ctcttgtcaa<br>661 catctcatct tttgacccaa tcttattcat ttaataagag gtctcattca tttgcatgga<br>721 aaaatgctca ttgtatattg caaagtgaaa ataacgagtt gcaaaacagt gtatacatat<br>781 atgtgtgtat atatgtacac tttatttgta catttctatg tgacataatg caaaggaaag<br>841 tgtctgattt tattatacac caaaggttaa cagtgaatct ctgtgtgatc tctttttttt<br>901 tctttttgcc tatctgcatc ttctcacttg ccaaaaaatg aatatatgtt tatgtgtgta<br>961 tattacttgt gtcacaaaaa accctaaagt agacagtaaa agaacttgtc aatcgccttt<br>1021 ggaaggcaat gaaacactta ataaactctc aataacagaa gcgtaaaaat gaaatgtaaa<br>1081 cctccaatta cctctggatc tcttagccag agtaataaac tggtaattat tacaggtaaa<br>1141 aaaaaaaaaa aaaaaaaaaa aaaa |
| PIGR (NM_ 002644.2) | 2.05E+08 (-strand) | 1 agagtttcag ttttggcagc agcgtccagt gccctgccag tagctcctag agaggcaggg<br>61 gttaccaact ggccagcagg ctgtgtccct gaagtcagat caacgggaga gaaggaagtg<br>121 gctaaaacat tgcacaggag aagtcggcct gagtggtgcg gcgctcggga cccaccagca<br>181 atgctgctct tcgtgctcac ctgcctgctg gcggtcttcc cagccatctc cacgaagagt<br>241 cccatatttg gtcccgagga ggtgaatagt gtggaaggta actcagtgtc catcacgtgc<br>301 tactacccac ccacctctgt caaccggcac acccggaagt actggtgccg gcagggagct<br>361 agaggtggct gcataaccct catctcctcg gagggctacg tctccagcaa atatgcaggc<br>421 agggctaacc tcaccaactt cccggagaac ggcacatttg tggtgaacat tgcccagctg<br>481 agccaggatg actccgggcg ctacaagtgt ggcctgggca tcaatagccg aggcctgtcc |

(continued)

| Gene name (GenBank accession number) | CpG co ordinates | mRNA/Coding Sequence* |
|---|---|---|
| | | 541 tttgatgtca gcctggaggt cagccagggt cctgggctcc taaatgacac taaagtctac<br>601 acagtggacc tgggcagaac ggtgaccatc aactgccctt tcaagactga gaatgctcaa<br>661 aagaggaagt ccttgtacaa gcagataggc ctgtaccctg tgctggtcat cgactccagt<br>721 ggttatgtaa atcccaacta tacaggaaga atacgccttg atattcaggg tactggccag<br>781 ttactgttca gcgttgtcat caaccaactc aggctcagcg atgctgggca gtatctctgc<br>841 caggctgggg atgattccaa tagtaataag aagaatgctg acctccaagt gctaaagccc<br>901 gagcccgagc tggtttatga agacctgagg ggctcagtga ccttccactg tgccctgggc<br>961 cctgaggtgg caaacgtggc caaatttctg tgccgacaga gcagtgggga aaactgtgac<br>1021 gtggtcgtca acaccctggg gaagagggcc ccagcctttg agggcaggat cctgctcaac<br>1081 ccccaggaca aggatggctc attcagtgtg gtgatcacag gcctgaggaa ggaggatgca<br>1141 gggcgctacc tgtgtggagc ccattcggat ggtcagctgc aggaaggctc gcctatccag<br>1201 gcctggcaac tcttcgtcaa tgaggagtcc acgattcccc gcagccccac tgtggtgaag<br>1261 ggggtggcag gaggctctgt ggccgtgctc tgcccctaca accgtaagga aagcaaaagc<br>1321 atcaagtact ggtgtctctg ggaaggggcc cagaatggcc gctgccccct gctggtggac<br>1381 agcgaggggt gggttaaggc ccagtacgag ggccgcctct ccctgctgga ggagccaggc<br>1441 aacggcacct tcactgtcat cctcaaccag ctcaccagcc gggacgccgg cttctactgg<br>1501 tgtctgacca acggcgatac tctctggagg accaccgtgg agatcaagat tatcgaagga<br>1561 gaaccaaacc tcaaggtacc agggaatgtc acggctgtgc tgggagagac tctcaaggtc<br>1621 ccctgtcact ttccatgcaa attctcctcg tacgagaaat actggtgcaa gtggaataac<br>1681 acgggctgcc aggccctgcc cagccaagac gaaggcccca gcaaggcctt cgtgaactgt<br>1741 gacgagaaca gccggcttgt ctccctgacc ctgaacctgg tgaccagggc tgatgagggc<br>1801 tggtactggt gtggagtgaa gcagggccac ttctatggag agactgcagc cgtctatgtg<br>1861 gcagttgaag agaggaaggc agcggggtcc cgcgatgtca gcctagcgaa ggcagacgct<br>1921 gctcctgatg agaaggtgct agactctggt tttcgggaga ttgagaacaa agccattcag<br>1981 gatcccaggc tttttgcaga ggaaaaggcg gtggcagata caagagatca agccgatggg<br>2041 agcagagcat ctgtggattc cggcagctct gaggaacaag gtggaagctc cagagcgctg<br>2101 gtctccaccc tggtgcccct gggcctggtg ctggcagtgg gagccgtggc tgtggggtg<br>2161 gccagagccc ggcacaggaa gaacgtcgac cgagtttcaa tcagaagcta caggacagac<br>2221 attagcatgt cagacttcga gaactccagg gaatttggag ccaatgacaa catgggagcc<br>2281 tcttcgatca ctcaggagac atccctcgga ggaaaagaag agtttgttgc caccactgag<br>2341 agcaccacag agaccaaaga acccaagaag gcaaaaaggt catccaagga ggaagccgag<br>2401 atggcctaca aagacttcct gctccagtcc agcaccgtgg ccgccgaggc ccaggacggc<br>2461 ccccaggaag cctagacggt gtcgccgcct gctccctgca cccatgacaa tcaccttcag |

(continued)

| Gene name (GenBank accession number) | CpG co ordinates | mRNA/Coding Sequence* |
|---|---|---|
| | | 2521 aatcatgtcg atcctggggc cctcagctcc tggggacccc actccctgct ctaacacctg<br>2581 cctaggtttt tcctactgtc ctcagaggcg tgctggtccc ctcctcagtg acatcaaagc<br>2641 ctggcctaat tgttcctatt ggggatgagg gtggcatgag gaggtcccac ttgcaacttc<br>2701 tttctgttga gagaacctca ggtacggaga agaatagagg tcctcatggg tcccttgaag<br>2761 gaagagggac cagggtggga gagctgattg cagaaaggag agacgtgcag cgcccctctg<br>2821 cacccttatc atgggatgtc aacagaattt ttccctccac tccatccctc cctcccgtcc<br>2881 ttcccctctt cttctttcct tccatcaaaa gatgtatttg aattcatact agaattcagg<br>2941 tgctttgcta gatgctgtga caggtatgcc accaacactg ctcacagcct ttctgaggac<br>3001 accagtgaaa gaagccacag ctcttcttgg cgtatttata ctcactgagt cttaactttt<br>3061 caccaggggt gctcacctct gcccctattg ggagaggtca taaaatgtct cgagtcctaa<br>3121 ggccttaggg gtcatgtatg atgagcatac acacaggtaa ttataaaccc acattcttac<br>3181 catttcacac ataagaaaat tgaggtttgg aagagtgaag cgtttttctt tttcttttt<br>3241 tttttgaga cggagtctct cactgtcgcc caggctggag tgcagtggcg caatctcggc<br>3301 tcactgcaac ctccgcctcc caggttgaca ccattctcct gcctcaccct cccaagtagc<br>3361 tgggactaca ggcgcctgcc agcacgcctg gctaattttt tgtattttta gtagagacag<br>3421 ggtttcaccg tgttagccag gatggtctcg atctcctgac ctcgtgatcc gcctgcctct<br>3481 gcctcccaaa gtgctgggat tacaggcgtg agccaccgcg tccggcctct tttttcttt<br>3541 tcttttttt gagacaaagt ctcactgtgt cacccagact ggaatgcagt gacacaatct<br>3601 cggctcactg aaacctctgc cttccaggtt caagctattc tcatgcctca gcctctcaag<br>3661 tagctgggac tacagatgtg ggccaccatg tctggctaat tttttttttt tttttttttt<br>3721 tttgtagaga cagggtttcg ccatgttgac gagactggtc tcgaactcct ggcctcaagt<br>3781 gatctgccgc ctcagcttct caaagtactg ggattatata ggcatgagcc actgagcctg<br>3841 gccctgaagc gtttttctca aaggccctca gtgagataaa ttagatttgg catctcctgt<br>3901 cctgggccag ggatctctct acaagagccc ctgcccctct gttggaggca cagttttaga<br>3961 ataaggagga ggagggagaa gagaaaatgt aaaggaggga gatctttccc aggccgcacc<br>4021 atttctgtca ctcacatgga cccaagataa aagaatggcc aaaccctcac aacccctgat<br>4081 gtttgaagag ttccaagttg aagggaaaca aagaagtgtt tgatggtgcc agagaggggc<br>4141 tgctctccag aaagctaaaa tttaatttct tttttcctct gagttctgta cttcaaccag<br>4201 cctacaagct ggcacttgct aacaaatcag aaatatgaca attaatgatt aaagactgtg<br>4261 attgcc |
| GJA12 (NM_ 020435.2) | 2.26E+08 (+strand) | 1 ggggaacaat ggggcccttg agggcccctc ctccagcccc cattgtgctt ggtggtgaga<br>61 ggtggccctg gctcggccac acaccctcgg ggaggaccag catccaagca ggtggaaggg |

(continued)

| Gene name (GenBank accession number) | CpG co ordinates | mRNA/Coding Sequence* |
|---|---|---|
| | | 121 ctctgaggga gactggaatt ttctggcctg gagaaggacc cgcccgcccg cccctatgac<br>181 caacatgagc tggagcttcc tgacgcggct gctggaggag atccacaacc actccacctt<br>241 cgtgggcaag gtgtggctca cggtgctggt ggtcttccgc atcgtgctga cggctgtggg<br>301 cggcgaggcc atctactcgg acgagcaggc caagttcact tgcaacacgc ggcagccagg<br>361 ctgcgacaac gtctgctatg acgccttcgc gcccctgtcg cacgtgcgct tctgggtctt<br>421 ccagattgtg gtcatctcca cgccctcggt catgtacctg ggctacgccg tgcaccgcct<br>481 ggcccgtgcg tctgagcagg agcggcgccg cgccctccgc cgccgcccgg ggccacgccg<br>541 cgcgccccga gcgcacctgc cgcccccgca cgccggctgg cctgagcccg ccgacctggg<br>601 cgaggaggag cccatgctgg gcctgggcga ggaggaggag gaggaggaga cgggggcagc<br>661 cgagggcgcc ggcgaggaag cggaggaggc aggcgcggag gaggcgtgca ctaaggcggt<br>721 cggcgctgac ggcaaggcgg cagggacccc gggcccgacc gggcaacacg atgggcggag<br>781 gcgcatccag cgggagggcc tgatgcgcgt gtacgtggcc cagctggtgg ccagggcagc<br>841 tttcgaggtg gccttcctgg tgggccagta cctgctgtac ggcttcgagg tgcgaccgtt<br>901 ctttccctgc agccgccagc cctgcccgca cgtggtggac tgcttcgtgt cgcgccctac<br>961 tgaaaagacg gtcttcctgc tggttatgta cgtggtcagc tgcctgtgcc tgctgctcaa<br>1021 cctctgtgag atggcccacc tgggcttggg cagcgcgcag gacgcggtgc gcggccgccg<br>1081 cggcccccg gcctccgccc ccgcccccgc gccgcggccc ccgccctgcg ccttccctgc<br>1141 ggcggccgct ggcttggcct gcccgcccga ctacagcctg gtggtgcggg cggccgagcg<br>1201 cgctcgggcg catgaccaga acctggcaaa cctggccctg caggcgctgc gcgacggggc<br>1261 agcggctggg gaccgcgacc gggacagttc gccgtgcgtc ggcctccctg cggcctcccg<br>1321 gggcccccc agagcaggcg cccccgcgtc ccggacgggc agtgctacct ctgcgggcac<br>1381 tgtcggggag cagggccggc ccggcaccca cgagcggcca ggagccaagc ccaggctgg<br>1441 ctccgagaag ggcagtgcca gcagcaggga cgggaagacc accgtgtgga tctgagggcg<br>1501 ctggcttgcg agctgggcca gggaggagga gggttggggg gctccggtgg aaacctgcga<br>1561 ccccttctcc tcagccttct ccttagccgg tggcctcagg cagactctgc ccagaggggc<br>1621 agccaggctg ctcagggaag gggctgaaag cggcagagga gtgccctggc ttggtcacca<br>1681 ctggggccaa ggtggggtgg agagaggcct aggagccaga aagggccctc tgctgtggtc<br>1741 tgaaccccag ggggagtggg gcattgactc caccccctgtc ctgagctgga ataggtcctc<br>1801 tgggatgcca gctctcccct ttgtgcttcc ctgcagcaac ccatggaggg cccagggtgc<br>1861 ctggtatggg catcagttgg tgggggtgcg ggggtgcgtg tccccattcc ctgcaacagc<br>1921 aaatggggct ccttcttcag ccctcccctt cccagcccca aactgagaca gactgggagc<br>1981 tgggagcctg ggtggacag gaccataccc tctttgagct tctgcgatgc cggccttccg<br>2041 ttcctctggg aggcttgaag ttctgcaaag atgttgatat gccttgcagc ttggacccaa |
| | | 2101 tgggtggtgg tcagggcctg gggcttggc catgctgggg gaatggggct ctgggttcct<br>2161 gcctgtggcc tgtctgtcct cctccctaat tcagacccag cctcaagagg aaagggagta<br>2221 aaataaaact aacttgttta taaaaaaaaa aaaaaaaaa |

(continued)

| Gene name (GenBank accession number) | CpG co ordinates | mRNA/Coding Sequence* |
|---|---|---|
| RIN2 (NM_ 018993.2) | 19817644 (+strand) | 1 gagtccccgg cgtgcagtgg agcctcgctg ggggaaatga cagcttggac catgggcgcc<br>61 cgcggtctgg acaagcgagg aagtttcttt aagctcattg acacaattgc ctcggagatc<br>121 ggagaactga aacaggagat ggtgcggaca gatgtcaacc tggaaaatgg cctggaaccc<br>181 gctgaaaccc acagcatggt aagacacaag gatggtggct attccgagga agaggacgtg<br>241 aagacctgtg cccgggactc aggctatgac agcctctcca acaggctcag catcttggac<br>301 cggctcctcc acacccaccc catatggctg cagctgagtc tgagtgagga ggaggcagca<br>361 gaggtcctgc aggcccagcc tccggggatc ttcctggttc ataaatctac caagatgcag<br>421 aagaaagtcc tctccctccg cctgccctgt gaatttgggg ccccactcaa ggaatttgcc<br>481 ataaaggaaa gcacatacac cttttccctg gaaggctcag gaatcagttt cgcagattta<br>541 ttccggctca ttgctttcta ctgcatcagc agggatgttc taccatttac cttgaagttg<br>601 ccttatgcca tttcaacagc caagtcggag gctcagcttg aagaactggc ccagatggga<br>661 ctaaatttct ggagctcccc agctgacagc aaacccccga accttccacc tccccatagg<br>721 cctctttcct ccgacggtgt ctgtcctgcc tccctgcgtc agctctgcct tataaatgga<br>781 gtgcattcta tcaaaaccag gacgccttca gagctggagt gcagccagac caacggggcc<br>841 ctgtgcttta ttaatcccct tttcttgaaa gtgcacagcc aggacctcag tggaggcctg<br>901 aaacggccga gcacaaggac tcccaacgcg aatggcacgg agcggactcg gtcccccca<br>961 cccaggcccc cgccacccgc tattaatagt ctccacacaa gccctcggct ggccaggact<br>1021 gaaacccaga cgagcatgcc agaaacagtc aaccataaca aacatgggaa cgtagctctg<br>1081 cctggaacga aaccaactcc catccctcca ccccggctga agaagcaggc ttcttttctg<br>1141 gaagcagagg gcggtgcaaa gaccttgagc ggcggccggc cgggcgcagg cccggagctg<br>1201 gagctgggca cagctggcag cccaggtggg gccccgcctg aggccgcccc gggggattgc<br>1261 acaagggccc cgccgcccag ctctgaatca cggcccccgt gccatggagg ccggcagcgg<br>1321 ctgagcgaca tgagcatttc tacttcctcc tccgactcgc tggagttcga ccggagcatg<br>1381 cctctgtttg gctacgaggc ggacaccaac agcagcctgg aggactacga gggggaaagt<br>1441 gaccaagaga ccatggcgcc ccccatcaag tccaaaaaga aaaggagcag ctccttcgtg<br>1501 ctgcccaagc tcgtcaagtc ccagctgcag aaggtgagcg ggtgttcag ctccttcatg<br>1561 accccggaga agcggatggt ccgcaggatc gccgagcttt cccgggacaa atgcacctac<br>1621 ttcgggtgct tagtgcagga ctacgtgagc ttcctgcagg agaacaagga gtgccacgtg<br>1681 tccagcaccg acatgctgca gaccatccgg cagttcatga cccaggtcaa gaactatttg |

(continued)

| Gene name (GenBank accession number) | CpG co ordinates | mRNA/Coding Sequence* |
|---|---|---|
| | | 1741 tctcagagct cggagctgga ccccccatc gagtcgctga tccctgaaga ccaaatagat<br>1801 gtggtgctgg aaaaagccat gcacaagtgc atcttgaagc ccctcaaggg gcacgtggag<br>1861 gccatgctga aggactttca catggccgat ggctcatgga agcaactcaa ggagaacctg<br>1921 cagcttgtgc ggcagaggaa tccgcaggag ctgggggtct tcgccccgac ccctgatttt<br>1981 gtggatgtgg agaaaatcaa agtcaagttc atgaccatgc agaagatgta ttcgccggaa<br>2041 aagaaggtca tgctgctgct gcgggtctgc aagctcattt acacggtcat ggagaacaac<br>2101 tcagggagga tgtatggcgc tgatgacttc ttgccagtcc tgacctatgt catagcccag<br>2161 tgtgacatgc ttgaattgga cactgaaatc gagtacatga tggagctcct agacccatcg<br>2221 ctgttacatg gagaaggagg ctattacttg acaagcgcat atggagcact ttctctgata<br>2281 aagaatttcc aagaagaaca agcagcgcga ctgctcagct cagaaaccag agacaccctg<br>2341 aggcagtggc acaaacggag aaccaccaac cggaccatcc cctctgtgga cgacttccag<br>2401 aattacctcc gagttgcatt tcaggaggtc aacagtggtt gcacaggaaa gaccctcctt<br>2461 gtgagacctt acatcaccac tgaggatgtg tgtcagatct gcgctgagaa gttcaaggtg<br>2521 ggggaccctg aggagtacag cctctttctc ttcgttgacg agacatggca gcagctggca<br>2581 gaggacactt accctcaaaa aatcaaggcg gagctgcaca gccgaccaca gccccacatc<br>2641 ttccactttg tctacaaacg catcaagaac gatccttatg gcatcatttt ccagaacggg<br>2701 gaagaagacc tcaccacctc ctagaagaca ggcgggactt cccagtggtg catccaaagg<br>2761 ggagctggaa gccttgcctt cccgcttcta catgcttgag cttgaaaagc agtcacctcc<br>2821 tcggggaccc ctcagtgtag tgactaagcc atccacaggc caactcggcc aagggcaact<br>2881 ttagccacgc aaggtagctg aggtttgtga aacagtagga ttctcttttg gcaatggaga<br>2941 attgcatctg atggttcaag tgtcctgaga ttgtttgcta cctacccca gtcaggttct<br>3001 aggttggctt acaggtatgt atatgtgcag aagaaacact taagatacaa gttcttttga<br>3061 attcaacagc agatgcttgc gatgcagtgc gtcaggtgat tctcactcct gtggatggct<br>3121 tcatccctgc cttccttcct ttctttttcc tttttttttt tttttttttt ttttttacaa<br>3181 agagccttca tgtttttata tatttcatag aaatttttat agcagttgca ggtaaactgt<br>3241 caggattggt tttaaaatat ttttgtaact ttaaaatatt ctataattat gcatgtgatt<br>3301 ttaacattta atattcaaaa ataaatctct tgctggattt gagagtattg catttttaaa<br>3361 gtctctcttc tgtaactgga tgttttggca actttgtggg gagagactgc tggatttctt<br>3421 aaagcaacgt attcctgaca ctggccacag aatgcctttg gaaatcggat gtactgttct<br>3481 cttgttcacg tttagtggtg ttttgctgtt ttgtttttta aacaaatgat gctgagaata<br>3541 aggagagaaa tgaatgtaga gagaggtaga gagagaaata tgaactctaa caaaggactg<br>3601 aggagtgcag tctgctggtt caggctcttc aaaagatgta gaaaaagaga tagaaggaac<br>3661 cacctatgct taaaatactg taaatatgca gtgaggtttg gcaaaatcta ttccatgtgt |
| | | 3721 gatttgcttg tagaaacaat tttgaaagcc ccttgaggaa aataaaaatc aagaagaaca<br>3781 cttttctccc ttttccatac aaattaaaac ttaacagcat caaattattg ggaccagaaa<br>3841 ccaagtaatg tataatgtgg cttttgttga gttaaataag atgctatata atggagaaga<br>3901 atttgaaaat gcacaaaaaa atcaatctac attatcagaa cctgcagtga aattaaactt<br>3961 atgttaaata aaaccagttt gcaggtgcac aaactatgag ggtcttgtat ccacgtaaca<br>4021 caggtagtta caaaaacatg ttattgtact gtgtaaagat gcatagtcat ctcatttggt<br>4081 tggctttgta ccttgtacct tttttagcct tggcttttgt tgaactagaa ccctcagcac<br>4141 atactgtgtt gtacttttgt aaatgatttt ttaaatggaa ttttgcacat aatacattgt<br>4201 aatactgtat gataatcatg tgtgaaaata atttttgaaa tatcaaaaaa aaaaaaaaa |

(continued)

| Gene name (GenBank accession number) | CpG co ordinates | mRNA/Coding Sequence* |
|---|---|---|
| RGN (NM_ 004683.4) | 46822773 (+ strand) | 1 gtgcccgagc caggccggcc tccccgcccc ctccctggaa aggaaaggcc ccggcgacaa<br>61 cagagccaga cccgctcatc ccgatctccc agaaggcgac tgacagctga ctgccagaag<br>121 gagatcgcgc caggagactg actgctctgt gcccacccgg ggacccgggc ccgttcagcc<br>181 gggctggctg gtgcgccctc tgcaaagcct gcgccaggga ggaggcaggc tcaaccttca<br>241 gattcccagg gcctctctgt cgctgtcgcc gtcgccgtcg cccgaggtcc cagcggctct<br>301 accagattgt tgtggaggcc tctcacccgc acagatctcc cctgcgacca tgtcttccat<br>361 taagattgag tgtgttttgc cagagaactg ccggtgtggt gagtctccag tatgggagga<br>421 agtgtccaac tctctgctct ttgtagacat tcctgcaaaa aaggtttgcc ggtgggattc<br>481 attcaccaag caagtacagc gagtgaccat ggatgcccca gtcagctccg tggctcttcg<br>541 ccagtcggga ggctatgttg ccaccattgg aacaaagttc tgtgctttga actggaaaga<br>601 acaatcagca gttgtcttgg ccacggtgga taacgacaag aaaaacaatc gcttcaatga<br>661 tgggaaggtg gatcccgccg ggaggtactt tgctggcacc atggctgagg aaacagctcc<br>721 agcagttctt gagcggcacc aggggccct gtactccctc tttcctgatc accacgtgaa<br>781 aaagtacttt gaccaggtgg acatttccaa tggtttggat tggtcgctag accacaaaat<br>841 cttctattac attgacagcc tgtcctactc cgtggatgcc tttgactatg acctgcagac<br>901 aggacagatc tccaaccgca gaagtgttta caagctagaa aaggaagaac aaatcccaga<br>961 tggaatgtgt attgatgctg aggggaagct ctgggtggcc tgttacaatg gaggaagagt<br>1021 gattcgttta gatcctgtga cagggaaaag acttcaaact gtgaagttgc ctgttgataa<br>1081 aacaacttca tgctgctttg gagggaagaa ttactctgaa atgtatgtga cctgcgcccg<br>1141 ggatgggatg gaccccgagg gtcttttgag gcaacctgaa gctggtggaa ttttcaagat<br>1201 aactggtctg gggtcaaag gaattgctcc ctactcctat gcgggatgag gacaggtctt<br>1261 ctttcctgcc agagggagct ctgaagacaa ctagagaatt ctgggcctga aatttcaatc<br>1321 tagttagaaa gaaaaatgag gcaatgattt tattaacagc gttaagtttt aatttacaac |
| | | 1381 ttttaaaagg cagagcattt ttaacaaggg gtgacaggtg gttttgataa cacacttata<br>1441 aggctttctg taaaaggtac tatagaaggg cgaagaatcg ttcaactgtc aatcagcctc<br>1501 ttgattcttt gtaaattgcc agggtgggtg ggtacatatc tcttcttgat tctgcatttc<br>1561 atacttaact atattaaagc ttcaaggaac aataaatagt aacctggtaa tgaccaaaaa<br>1621 aaaaaaaaaa aaaaa |
| TCEAL7 (NM_ 152278.1) | 102471609 (+ strand) | 1 gggggtacca gctccttact gccctgcaga caagcgtgcc gtgcgtgctt gtggccaagg<br>61 gaaggaagag ctggttgatc cacagatagc tccttcctcc ccgccccttc ctttttgttt<br>121 ggaggtccca ggatctgtgt tcacagacat ctgggggaag aaaaggagca ggaaactacc<br>181 ccgcacagag ttaagcagga aacaacaaca acatcatgca aaaaccctgc aaagaaaacg<br>241 aaggaaagcc aaagtgcagc gtgccaaaga gggaggaaaa acgcccgtat ggagaatttg<br>301 aacgccagca aacagaaggg aattttagac agaggctgct tcagtctctc gaagaattta<br>361 aagaggacat agactatagg cattttaaag atgaagaaat gacaagggag ggagatgaga<br>421 tggaaaggtg tttggaagag ataaggggtc tgagaaagaa atttagggct ctgcattcta<br>481 accataggca ttctcgggac cgtccttatc ccatttaatt aatttctctg acaattcaat<br>541 tattttctgt tattaatgtt gccactgctt tctgtttgtc tgcactttct tgataaatat<br>601 ttgctatcgt tttactccag tcattcgatg ttgctgagat ttacatatga ctcttgtcaa<br>661 catctcatct tttgacccaa tcttattcat ttaataagag gtctcattca tttgcatgga<br>721 aaaatgctca ttgtatattg caaagtgaaa ataacgagtt gcaaaacagt gtatacatat<br>781 atgtgtgtat atatgtacac tttatttgta catttctatg tgacataatg caaaggaaag<br>841 tgtctgattt tattatacac caaaggttaa cagtgaatct ctgtgtgatc tctttttttt<br>901 tctttttgcc tatctgcatc ttctcacttg ccaaaaaatg aatatatgtt tatgtgtgta<br>961 tattacttgt gtcacaaaaa accctaaagt agacagtaaa agaacttgtc aatcgccttt<br>1021 ggaaggcaat gaaacactta ataaactctc aataacagaa gcgtaaaaat gaaatgtaaa<br>1081 cctccaatta cctctggatc tcttagccag agtaataaac tggtaattat tacaggtaaa<br>1141 aaaaaaaaaa aaaaaaaaaa aaaa |
| * The coding sequence (mRNA sequence) is provided for ease of reference. Clearly mRNAs are not typically methylated. The sequence which is assayed according to the present invention is suitably the DNA sequence. This is suitably the genomic sequence. The CpG co-ordinates for the addresses of interest on the DNA sequence are provided. The mRNA/coding sequences are provided for illustration only in case any further assistance is needed by the skilled operator in locating the sequences of interest. | | |

As the skilled person knows, the accession numbers above are absolute (dated) accession numbers. The database entries can be amended over time. Suitably the current database entry is used. The accession numbers for the current database entry are the same as above, but omitting the decimal point and any subsequent digits e.g. for SLC22A18 the absolute/dated accession number is NM_183233.1; the current entry is obtained using NM_183233 and so on.

Suitably the database for reference sequences is GenBank (National Center for Biotechnology Information, U.S. National Library of Medicine 8600 Rockville Pike, Bethesda MD, 20894 USA) and accession numbers provided relate to this unless otherwise apparent.

Suitably the database release referred to is 15 April 2013, NCBI-GenBank Release 195.0.

**Sample**

The sample may be from a subject. The subject is suitably a mammal, most suitably a human.

Suitably the methods do not involve actual collection of the sample. Suitably the sample is an *in vitro* sample.

Methods of the invention are suitably performed on an isolated sample from the subject being investigated. Thus, suitably the methods are methods which may be conducted in a laboratory setting without the need for the subject to be present. Suitably the methods are carried out *in vitro* i.e. suitably the methods are *in vitro* methods. Suitably the methods are extracorporeal methods.

Suitably the invention is applied to analysis of nucleic acids. Suitably, nucleic acid is prepared from the cells collected from the subject of interest. Suitably, the sample comprises nucleic acid. Suitably, the sample consists of nucleic acid. Suitably, the nucleic acid is DNA.

Suitably the sample comprises cells from the surface of a subject's upper intestinal tract. Suitably the sample consists of cells from the surface of a subject's upper intestinal tract. Suitably the sample comprises cells from the surface of a subject's oesophagus. Suitably the sample consists of cells from the surface of a subject's oesophagus.

Suitably the sample is an *in vitro* sample.
Suitably the sample is an extra corporeal sample.

Suitably the sample is from a subject having Barrett's Esophagus.

Suitably the sample comprises material taken from the region of the Barrett's Esophagus. Suitably the sample comprises materialtaken from the Barrett's Esophagus segment itself.

Suitably the sample is a biopsy.

Suitably the sample does not comprise formalin fixed paraffin embedded (FFPE) material. Pyrosequencing can be problematic on this type of material. Thus suitably the sample is such that pyrosequencing is possible.

Suitably the sample comprises fresh, chilled or frozen biopsy material. Suitably the sample comprises frozen biopsymaterial.

Suitably the biopsy material is endoscopically collected. Suitably the biopsy is a standard 'pinch-type' biopsy. Suitably this is collected in a standard forceps-pinch technique.

In one embodiment sampling the cellular surface of the upper intestinal tract such as the oesophagus may comprise the steps of

(i) introducing a swallowable device comprising abrasive material capable of collecting cells from the surface of the oesophagus into the subject,
(ii) retrieving said device by with drawal through the oesophagus, and
(iii) collecting the cells from the device.

Suitably step (i) comprises introducing a swallowable device comprising abrasive material capable of collecting cells from the surface of the oesophagus into the subject's stomach. Suitably said cell collection device comprises a capsule sponge. Suitably the device is a capsule sponge as described in WO2007/045896 and/or as described in WO2011/058316. These two documents are referred to specifically for the description of the structure and/or construction of the cell collection devices (capsule sponges). Suitably said cell collection device comprises withdrawal means such as string. In one embodiment, the invention involves the sampling of the cells from the surface of the oesophagus using a swallowable abrasive material, which material is retrieved from the patient and from which the cells are subsequently separated for analysis. Suitably the majority of the surface of the oesophagus is sampled, more suitably substantially the entire surface of the oesophagus is sampled, most suitably the entire surface. Suitably the whole internal surface of the oesophagus ie. the complete inner lumen is sampled. In this embodiment abrasive is meant that the material is capable of removing cells from the internal surface of the oesophagus. Clearly, since this is meant for use in a subject's oesophagus, 'abrasive' must be interpreted in the light of the application. In the context of the present invention the term 'abrasive' has the meaning given above, which can be tested by passing the material through the oesophagus in an appropriate amount/configuration and examining it to determine whether cells have been removed from the oesophagus. Suitably the swallowable abrasive material is expandable. In this embodiment, suitably the abrasive material is of a smaller size when swallowed than when withdrawn. An expandable material may be simply a resilient material compressed such that when released from compression it will expand again back to a size approximating its uncompressed size. Alternatively it may be a material which expands eg. upon taking up aqueous fluid to a final size exceeding its original size.

**Assay of Methylation Status**

Any suitable technique known in the art may be used to assay methylation of the genes of interest.

For example pyrosequencing may be used. Further details are found in the examples section.

For example MSP (Methyl-specific PCR) may be used.

For example the MethyLight assay may be used (Eads, C.A. et al. MethyLight: a high-throughput assay to measure DNA methylation. Nucleic Acids Res 28, E32 (2000)). Kits for this type of assay are commercially available such as from Qiagen Inc., Hilden, Germany.

Most suitably the technique is suitable for use on frozen sample material.

Methylation status is suitably scored.

Methylation status is suitably scored in a binary 'present' or 'absent' manner.

Methylation status is more suitably scored by determining the level of methylation in the gene of interest.

Methylation status is most suitably scored by comparing the level of methylation in the gene of interest with a reference standard.

Suitably the reference standard comprises an esophagal sample from a subject who does not have esophagal dysplasia such as esophagal high grade dysplasia.

Suitably the reference standard comprises an esophagal sample from a subject who does not have esophagal adenocarcinoma.

Suitably the reference standard comprises an esophagal sample from a subject who does not have esophagal dysplasia such as esophagal high grade dysplasia, and does not have esophagal adenocarcinoma.

As will be apparent from the disclosure herein, the skilled operator working the invention may choose different methylation cut-offs depending on the specificity/sensitivity desired. Broadly speaking, the higher the methylation cut-off, the more stringent the method (and the higher the specificity/sensitivity values).

Most suitably methylation status is scored by determining the level of methylation in the gene of interest and comparing the level of methylation in the gene of interest with a reference standard, which reference standard is most suitably as shown in supplementary table 7 as 'methylation cut-off'. Suitably this is done on a gene-by-gene basis. Suitably a methylation level matching or exceeding the 'methylation cut-off' is scored as 'methylated'. Suitably a methylation level lower than the 'methylation cut-off' is scored as 'not methylated'.

In more detail, it is an advantage of the invention that the methylation cut-offs can be chosen to specifically provide for the needs of the operator regarding sensitivity and/or specificity. The table below presents alternatives.

| Genes | cut-off | sensitivity | specificity |
|---|---|---|---|
| ***GJA12*** | 35.91-62.37 | 71%-99% | 72%-97% |
| ***SLC22A18*** | 43.54-59.24 | 70%-95% | 71%-97% |
| ***PIGR*** | 50.48-76.08 | 72%-95% | 72%-100% |
| ***RIN2*** | 31.61-45.02 | 70%-93% | 72%-97% |
| ***RGN* (males only)** | 15.27-23.82 | 70%-88% | 72%-88% |
| ***TCFAL7*** | 56.03-58.54 | 71%-73% | 72%-84% |

The lower cut-off gives the higher sensitivity and vice versa. For example, choosing a cut-off of 35.91 for GJA12 provides maximum sensitivity of 99%. Choosing a cut-off of 62.37 for GJA12 provides maximum specificity of 97% and so on.

Intermediate values may be chosen according to need.

Examples of intermediate values which may be chosen are provided below.

Individual cut-offs/sensitivities/specificities may be chosen for each gene in combinations according to the 6 tables presented below (one table of options per gene).

| PIGR cut-off | PIGR sensiti vity | PIGR specifi city | RIN2 cut-off | RIN2 sensiti vity | RIN2 specifi city | SLC22A18 cut-off | SLC22A1 8 sensitivi ty | SLC22 A18 specifi city |
|---|---|---|---|---|---|---|---|---|
| 20.0 | 100.0% | 0.0% | 4.8 | 100.0% | 0.0% | 14.0 | 100.0% | 0.0% |
| 22.3 | 100.0% | 3.1% | 8.0 | 100.0% | 3.1% | 17.0 | 100.0% | 3.2% |
| 24.0 | 100.0% | 6.3% | 11.7 | 100.0% | 6.3% | 20.0 | 100.0% | 6.5% |
| 24.9 | 100.0% | 9.4% | 15.8 | 100.0% | 9.4% | 21.5 | 100.0% | 9.7% |
| 26.5 | 100.0% | 12.5% | 18.5 | 100.0% | 12.5% | 22.9 | 100.0% | 12.9% |
| 28.3 | 100.0% | 15.6% | 18.9 | 100.0% | 15.6% | 24.4 | 100.0% | 16.1% |
| 29.3 | 100.0% | 18.8% | 19.6 | 98.6% | 15.6% | 25.5 | 100.0% | 19.4% |
| 29.8 | 100.0% | 21.9% | 20.3 | 98.6% | 18.8% | 26.5 | 100.0% | 22.6% |
| 30.1 | 100.0% | 25.0% | 20.9 | 98.6% | 21.9% | 27.1 | 100.0% | 25.8% |
| 31.1 | 100.0% | 28.1% | 21.5 | 98.6% | 25.0% | 27.6 | 100.0% | 29.0% |
| 32.3 | 100.0% | 31.3% | 22.1 | 98.6% | 28.1% | 28.0 | 100.0% | 32.3% |
| 34.1 | 100.0% | 34.4% | 22.7 | 98.6% | 31.3% | 29.2 | 100.0% | 35.5% |
| 36.0 | 100.0% | 37.5% | 23.0 | 98.6% | 34.4% | 30.5 | 100.0% | 38.7% |
| 36.7 | 100.0% | 40.6% | 23.2 | 97.3% | 34.4% | 30.9 | 100.0% | 41.9% |
| 37.9 | 100.0% | 43.8% | 23.5 | 97.3% | 37.5% | 31.7 | 100.0% | 45.2% |
| 39.2 | 100.0% | 46.9% | 23.8 | 97.3% | 40.6% | 33.1 | 100.0% | 48.4% |
| 40.8 | 100.0% | 50.0% | 23.9 | 95.9% | 40.6% | 34.1 | 100.0% | 51.6% |
| 42.1 | 100.0% | 53.1% | 24.0 | 95.9% | 43.8% | 35.0 | 100.0% | 54.8% |
| 43.0 | 100.0% | 56.3% | 24.2 | 94.6% | 43.8% | 35.8 | 100.0% | 58.1% |
| 44.1 | 98.7% | 56.3% | 24.4 | 94.6% | 46.9% | 36.2 | 100.0% | 61.3% |
| 45.1 | 98.7% | 59.4% | 24.5 | 94.6% | 50.0% | 36.4 | 98.6% | 61.3% |
| 45.5 | 98.7% | 62.5% | 24.8 | 94.6% | 53.1% | 36.7 | 97.3% | 61.3% |
| 45.9 | 98.7% | 65.6% | 25.7 | 94.6% | 56.3% | 38.5 | 97.3% | 64.5% |
| 46.2 | 98.7% | 68.8% | 27.1 | 94.6% | 59.4% | 40.2 | 95.9% | 64.5% |
| 47.3 | 97.3% | 68.8% | 27.9 | 94.6% | 62.5% | 40.7 | 94.5% | 64.5% |
| 48.4 | 96.0% | 68.8% | 28.5 | 94.6% | 65.6% | 41.9 | 94.5% | 67.7% |
| 49.2 | 94.7% | 68.8% | 30.0 | 94.6% | 68.8% | 43.5 | 94.5% | 71.0% |
| 50.5 | 94.7% | 71.9% | 31.2 | 93.2% | 68.8% | 44.2 | 94.5% | 74.2% |
| 51.5 | 94.7% | 75.0% | 31.6 | 93.2% | 71.9% | 44.5 | 94.5% | 77.4% |
| 52.5 | 93.3% | 75.0% | 32.4 | 91.9% | 71.9% | 45.0 | 93.2% | 77.4% |
| 54.9 | 93.3% | 78.1% | 32.9 | 90.5% | 71.9% | 45.8 | 91.8% | 77.4% |
| 56.8 | 93.3% | 81.3% | 33.7 | 89.2% | 71.9% | 46.4 | 91.8% | 80.6% |
| 56.9 | 93.3% | 84.4% | 34.7 | 89.2% | 75.0% | 46.8 | 91.8% | 83.9% |
| 57.8 | 93.3% | 87.5% | 35.5 | 89.2% | 78.1% | 48.0 | 90.4% | 83.9% |
| 59.7 | 90.7% | 87.5% | 36.1 | 89.2% | 81.3% | 49.3 | 90.4% | 87.1% |
| 61.8 | 90.7% | 90.6% | 36.3 | 87.8% | 81.3% | 49.7 | 89.0% | 87.1% |
| 63.1 | 89.3% | 90.6% | 36.4 | 86.5% | 81.3% | 50.3 | 87.7% | 87.1% |
| 64.1 | 88.0% | 90.6% | 36.5 | 86.5% | 84.4% | 50.7 | 86.3% | 87.1% |
| 64.8 | 88.0% | 93.8% | 36.7 | 85.1% | 87.5% | 50.9 | 84.9% | 87.1% |
| 65.8 | 86.7% | 93.8% | 37.0 | 83.8% | 87.5% | 51.1 | 84.9% | 90.3% |
| 67.6 | 85.3% | 93.8% | 37.9 | 83.8% | 90.6% | 51.2 | 83.6% | 90.3% |
| 70.0 | 84.0% | 93.8% | 38.8 | 82.4% | 90.6% | 51.9 | 82.2% | 90.3% |
| 71.8 | 82.7% | 93.8% | 39.5 | 81.1% | 90.6% | 52.6 | 82.2% | 93.5% |
| 72.1 | 81.3% | 93.8% | 39.8 | 79.7% | 90.6% | 52.8 | 80.8% | 93.5% |
| 72.3 | 80.0% | 93.8% | 39.9 | 78.4% | 90.6% | 53.5 | 79.5% | 93.5% |
| 72.8 | 78.7% | 93.8% | 40.0 | 77.0% | 90.6% | 54.5 | 78.1% | 93.5% |
| 73.3 | 77.3% | 93.8% | 40.5 | 75.7% | 90.6% | 55.0 | 76.7% | 93.5% |
| 73.9 | 76.0% | 93.8% | 41.9 | 74.3% | 90.6% | 55.5 | 76.7% | 96.8% |
| 74.4 | 74.7% | 93.8% | 43.1 | 73.0% | 90.6% | 56.0 | 75.3% | 96.8% |

(continued)

| PIGR cut-off | PIGR sensiti vity | PIGR specifi city | RIN2 cut-off | RIN2 sensiti vity | RIN2 specifi city | SLC22A18 cut-off | SLC22A1 8 sensitivi ty | SLC22 A18 specifi city |
|---|---|---|---|---|---|---|---|---|
| 74.8 | 74.7% | 96.9% | 43.5 | 71.6% | 90.6% | 56.4 | 74.0% | 96.8% |
| 75.3 | 73.3% | 96.9% | 43.9 | 70.3% | 90.6% | 57.3 | 72.6% | 96.8% |
| 75.8 | 72.0% | 96.9% | 44.2 | 70.3% | 93.8% | 58.4 | 71.2% | 96.8% |
| 76.1 | 72.0% | 100.0 % | 45.0 | 70.3% | 96.9% | 59.2 | 69.9% | 96.8% |
| 76.5 | 70.7% | 100.0 % | 45.7 | 68.9% | 96.9% | 59.8 | 68.5% | 96.8% |
| 78.0 | 69.3% | 100.0 % | 45.9 | 67.6% | 96.9% | 60.0 | 67.1 % | 96.8% |
| 79.2 | 68.0% | 100.0 % | 46.2 | 66.2% | 96.9% | 60.2 | 65.8% | 96.8% |
| 79.5 | 66.7% | 100.0 % | 47.0 | 64.9% | 96.9% | 60.7 | 64.4% | 96.8% |
| 79.9 | 65.3% | 100.0 % | 47.7 | 63.5% | 96.9% | 61.1 | 63.0% | 100.0 % |
| 81.7 | 64.0% | 100.0 % | 48.2 | 62.2% | 96.9% | 61.2 | 61.6% | 100.0 % |
| 84.0 | 62.7% | 100.0 % | 48.9 | 60.8% | 96.9% | 61.4 | 60.3% | 100.0 % |
| 84.6 | 61.3% | 100.0 % | 49.1 | 59.5% | 96.9% | 62.3 | 58.9% | 100.0 % |
| 85.1 | 60.0% | 100.0 % | 49.2 | 58.1% | 96.9% | 63.0 | 57.5% | 100.0 % |
| 85.7 | 58.7% | 100.0 % | 49.7 | 56.8% | 96.9% | 63.3 | 56.2% | 100.0 % |
| 86.3 | 57.3% | 100.0 % | 50.2 | 55.4% | 96.9% | 64.0 | 54.8% | 100.0 % |
| 86.7 | 56.0% | 100.0 % | 51.3 | 54.1% | 96.9% | 64.6 | 53.4% | 100.0 % |
| 86.7 | 54.7% | 100.0 % | 52.2 | 54.1% | 100.0 % | 64.8 | 52.1% | 100.0 % |
| 86.9 | 53.3% | 100.0 % | 52.6 | 52.7% | 100.0 % | 65.4 | 50.7% | 100.0 % |
| 87.3 | 50.7% | 100.0 % | 53.7 | 51.4% | 100.0 % | 66.3 | 49.3% | 100.0 % |
| 87.8 | 49.3% | 100.0 % | 54.8 | 50.0% | 100.0 % | 66.9 | 47.9% | 100.0 % |
| 88.0 | 48.0% | 100.0 % | 55.3 | 48.6% | 100.0 % | 67.2 | 46.6% | 100.0 % |
| 88.3 | 45.3% | 100.0 % | 55.7 | 47.3% | 100.0 % | 67.7 | 45.2% | 100.0 % |
| 88.6 | 44.0% | 100.0 % | 56.3 | 45.9% | 100.0 % | 68.0 | 43.8% | 100.0 % |
| 88.8 | 42.7% | 100.0 % | 57.3 | 44.6% | 100.0 % | 68.1 | 42.5% | 100.0 % |
| 88.9 | 41.3% | 100.0 % | 57.9 | 43.2% | 100.0 % | 68.6 | 41.1% | 100.0 % |
| 89.0 | 40.0% | 100.0 % | 58.7 | 41.9% | 100.0 % | 69.4 | 39.7% | 100.0 % |
| 89.2 | 38.7% | 100.0 % | 59.6 | 40.5% | 100.0 % | 69.8 | 38.4% | 100.0 % |
| 89.3 | 37.3% | 100.0 % | 59.9 | 39.2% | 100.0 % | 69.9 | 37.0% | 100.0 % |
| 89.4 | 36.0% | 100.0 % | 61.6 | 37.8% | 100.0 % | 71.0 | 35.6% | 100.0 % |
| 89.5 | 33.3% | 100.0 % | 63.6 | 36.5% | 100.0 % | 72.1 | 34.2% | 100.0 % |
| 89.6 | 32.0% | 100.0 % | 64.9 | 35.1% | 100.0 % | 72.2 | 32.9% | 100.0 % |
| 89.8 | 30.7% | 100.0 % | 66.3 | 33.8% | 100.0 % | 72.3 | 31.5% | 100.0 % |
| 90.0 | 28.0% | 100.0 % | 67.1 | 32.4% | 100.0 % | 72.8 | 30.1% | 100.0 % |
| 90.1 | 26.7% | 100.0 % | 67.3 | 31.1% | 100.0 % | 73.4 | 28.8% | 100.0 % |
| 90.1 | 25.3% | 100.0 % | 67.6 | 29.7% | 100.0 % | 73.8 | 27.4% | 100.0 % |
| 90.1 | 24.0% | 100.0 % | 67.8 | 28.4% | 100.0 % | 74.2 | 26.0% | 100.0 % |
| 90.2 | 22.7% | 100.0 % | 67.9 | 27.0% | 100.0 % | 74.5 | 24.7% | 100.0 % |
| 90.3 | 21.3% | 100.0 % | 68.3 | 25.7% | 100.0 % | 74.7 | 23.3% | 100.0 % |
| 90.7 | 18.7% | 100.0 % | 69.0 | 24.3% | 100.0 % | 75.5 | 21.9% | 100.0 % |
| 91.0 | 17.3% | 100.0 % | 69.7 | 23.0% | 100.0 % | 76.1 | 20.5% | 100.0 % |
| 91.0 | 16.0% | 100.0 % | 70.5 | 21.6% | 100.0 % | 76.9 | 19.2% | 100.0 % |
| 91.1 | 14.7% | 100.0 % | 71.4 | 20.3% | 100.0 % | 77.6 | 17.8% | 100.0 % |
| 91.2 | 13.3% | 100.0 % | 71.7 | 18.9% | 100.0 % | 78.4 | 16.4% | 100.0 % |
| 91.3 | 12.0% | 100.0 % | 72.4 | 17.6% | 100.0 % | 79.1 | 15.1% | 100.0 % |
| 91.4 | 10.7% | 100.0 % | 73.1 | 16.2% | 100.0 % | 79.8 | 13.7% | 100.0 % |
| 91.7 | 8.0% | 100.0 % | 73.5 | 14.9% | 100.0 % | 80.8 | 12.3% | 100.0 % |
| 92.0 | 6.7% | 100.0 % | 73.9 | 13.5% | 100.0 % | 81.2 | 9.6% | 100.0 % |
| 92.1 | 4.0% | 100.0 % | 74.0 | 12.2% | 100.0 % | 81.9 | 8.2% | 100.0 % |

(continued)

| PIGR cut-off | PIGR sensitivity | PIGR specificity | RIN2 cut-off | RIN2 sensitivity | RIN2 specificity | SLC22A18 cut-off | SLC22A18 sensitivity | SLC22A18 specificity |
|---|---|---|---|---|---|---|---|---|
| 92.2 | 2.7% | 100.0 % | 74.1 | 10.8% | 100.0 % | 82.9 | 5.5% | 100.0 % |
| 92.4 | 1.3% | 100.0 % | 74.3 | 9.5% | 100.0 % | 83.4 | 4.1% | 100.0 % |
| 93.5 | 0.0% | 100.0 % | 74.5 | 8.1% | 100.0 % | 85.0 | 2.7% | 100.0 % |
| | | | 74.8 | 6.8% | 100.0 % | 87.7 | 1.4% | 100.0 % |
| | | | 75.4 | 5.4% | 100.0 % | 90.0 | 0.0% | 100.0 % |
| | | | 77.7 | 4.1% | 100.0 % | | | |
| | | | 81.1 | 2.7% | 100.0 % | | | |
| | | | 85.4 | 1.4% | 100.0 % | | | |
| | | | 89.1 | 0.0% | 100.0 % | | | |

| GJA12 cut-off | GJA12 sensitivity | GJA12 specificity | RGN male cut-off | RGN male sensitivity | RGN male specificity | TCEAL7 cut-off | TCEAL7 sensitivity | TCEAL7 specificity |
|---|---|---|---|---|---|---|---|---|
| 10.9 | 100.0% | 0.0% | 0.0 | 100.0% | 0.0% | 25.7 | 100.0% | 0.0% |
| 12.4 | 100.0% | 3.1% | 2.5 | 100.0% | 4.0% | 27.2 | 100.0% | 3.1% |
| 13.5 | 100.0% | 6.3% | 4.6 | 100.0% | 8.0% | 28.7 | 98.7% | 3.1% |
| 14.3 | 100.0% | 9.4% | 5.9 | 100.0% | 12.0% | 29.7 | 98.7% | 6.3% |
| 14.6 | 100.0% | 12.5% | 6.9 | 100.0% | 16.0% | 30.9 | 98.7% | 9.4% |
| 15.4 | 100.0% | 15.6% | 7.3 | 98.0% | 16.0% | 33.2 | 97.3% | 9.4% |
| 17.1 | 100.0% | 18.8% | 7.5 | 96.0% | 16.0% | 36.2 | 97.3% | 12.5% |
| 18.4 | 100.0% | 21.9% | 7.7 | 94.0% | 16.0% | 38.1 | 97.3% | 15.6% |
| 19.0 | 100.0% | 25.0% | 7.8 | 94.0% | 20.0% | 38.9 | 96.0% | 15.6% |
| 20.7 | 100.0% | 28.1% | 7.9 | 94.0% | 24.0% | 40.4 | 94.7% | 15.6% |
| 22.2 | 100.0% | 31.3% | 8.1 | 94.0% | 28.0% | 41.2 | 93.3% | 15.6% |
| 23.5 | 100.0% | 34.4% | 8.4 | 94.0% | 32.0% | 41.3 | 93.3% | 18.8% |
| 24.6 | 100.0% | 37.5% | 8.8 | 94.0% | 36.0% | 41.5 | 93.3% | 21.9% |
| 25.4 | 100.0% | 40.6% | 9.0 | 94.0% | 40.0% | 41.8 | 93.3% | 25.0% |
| 26.5 | 100.0% | 43.8% | 9.7 | 94.0% | 44.0% | 42.2 | 93.3% | 28.1% |
| 27.4 | 100.0% | 46.9% | 10.9 | 94.0% | 48.0% | 42.7 | 92.0% | 28.1% |
| 27.8 | 100.0% | 50.0% | 11.6 | 94.0% | 52.0% | 43.5 | 90.7% | 28.1% |
| 28.7 | 100.0% | 53.1% | 11.9 | 94.0% | 56.0% | 44.4 | 90.7% | 31.3% |
| 29.8 | 100.0% | 56.3% | 12.2 | 94.0% | 60.0% | 45.3 | 90.7% | 34.4% |
| 30.4 | 100.0% | 59.4% | 12.9 | 94.0% | 64.0% | 46.0 | 88.0% | 34.4% |
| 31.1 | 98.7% | 59.4% | 13.3 | 92.0% | 64.0% | 47.0 | 86.7% | 34.4% |
| 32.6 | 98.7% | 62.5% | 13.6 | 90.0% | 64.0% | 48.1 | 86.7% | 37.5% |
| 33.9 | 98.7% | 65.6% | 14.3 | 90.0% | 68.0% | 48.2 | 85.3% | 37.5% |
| 34.8 | 98.7% | 68.8% | 14.9 | 88.0% | 68.0% | 48.2 | 84.0% | 37.5% |
| 35.9 | 98.7% | 71.9% | 15.3 | 88.0% | 72.0% | 48.4 | 82.7% | 37.5% |
| 36.7 | 97.3% | 71.9% | 15.6 | 86.0% | 72.0% | 48.9 | 82.7% | 40.6% |
| 37.1 | 97.3% | 75.0% | 16.8 | 84.0% | 72.0% | 49.2 | 82.7% | 43.8% |
| 38.0 | 97.3% | 78.1% | 17.8 | 82.0% | 72.0% | 49.3 | 81.3% | 43.8% |
| 40.1 | 97.3% | 81.3% | 18.0 | 82.0% | 76.0% | 49.6 | 81.3% | 46.9% |
| 41.7 | 96.0% | 81.3% | 18.6 | 82.0% | 80.0% | 50.0 | 81.3% | 50.0% |
| 42.2 | 96.0% | 84.4% | 19.3 | 80.0% | 80.0% | 50.3 | 80.0% | 50.0% |
| 44.0 | 96.0% | 87.5% | 20.1 | 78.0% | 80.0% | 51.2 | 80.0% | 53.1% |

(continued)

| GJA12 cut-off | GJA12 sensiti vity | GJA12 specifici ty | RGN male cut-off | RGN male sensiti vity | RGN male specificity | TCEAL7 cut-off | TCEAL7 sensitivity | TCEAL7 specifici ty |
|---|---|---|---|---|---|---|---|---|
| 47.0 | 96.0% | 90.6% | 20.8 | 78.0% | 84.0% | 52.0 | 80.0% | 56.3% |
| 49.0 | 94.7% | 90.6% | 21.0 | 76.0% | 84.0% | 52.2 | 80.0% | 59.4% |
| 50.2 | 94.7% | 93.8% | 21.4 | 74.0% | 84.0% | 52.3 | 78.7% | 59.4% |
| 51.7 | 94.7% | 96.9% | 22.1 | 72.0% | 84.0% | 52.5 | 78.7% | 62.5% |
| 52.9 | 93.3% | 96.9% | 23.0 | 70.0% | 84.0% | 53.2 | 78.7% | 65.6% |
| 54.4 | 92.0% | 96.9% | 23.8 | 70.0% | 88.0% | 54.1 | 78.7% | 68.8% |
| 56.1 | 90.7% | 96.9% | 24.2 | 68.0% | 88.0% | 55.0 | 77.3% | 68.8% |
| 56.5 | 89.3% | 96.9% | 24.4 | 66.0% | 88.0% | 55.4 | 76.0% | 68.8% |
| 56.7 | 88.0% | 96.9% | 25.2 | 64.0% | 88.0% | 55.7 | 74.7% | 68.8% |
| 56.9 | 86.7% | 96.9% | 26.5 | 64.0% | 92.0% | 55.7 | 73.3% | 68.8% |
| 57.2 | 85.3% | 96.9% | 27.3 | 62.0% | 92.0% | 56.0 | 73.3% | 71.9% |
| 57.6 | 84.0% | 96.9% | 27.6 | 60.0% | 92.0% | 56.5 | 72.0% | 71.9% |
| 57.9 | 82.7% | 96.9% | 27.7 | 58.0% | 92.0% | 56.7 | 72.0% | 75.0% |
| 58.2 | 81.3% | 96.9% | 28.5 | 56.0% | 92.0% | 57.2 | 72.0% | 78.1% |
| 58.5 | 80.0% | 96.9% | 31.1 | 56.0% | 96.0% | 57.9 | 72.0% | 81.3% |
| 59.4 | 78.7% | 96.9% | 33.4 | 54.0% | 96.0% | 58.2 | 70.7% | 81.3% |
| 60.5 | 77.3% | 96.9% | 34.0 | 52.0% | 96.0% | 58.5 | 70.7% | 84.4% |
| 61.0 | 76.0% | 96.9% | 34.3 | 50.0% | 96.0% | 59.0 | 69.3% | 84.4% |
| 61.1 | 74.7% | 96.9% | 34.5 | 48.0% | 96.0% | 59.3 | 68.0% | 84.4% |
| 61.2 | 73.3% | 96.9% | 34.9 | 46.0% | 96.0% | 59.5 | 66.7% | 84.4% |
| 61.7 | 72.0% | 96.9% | 35.3 | 44.0% | 96.0% | 59.7 | 65.3% | 84.4% |
| 62.4 | 70.7% | 96.9% | 35.5 | 42.0% | 96.0% | 60.4 | 64.0% | 84.4% |
| 63.4 | 69.3% | 96.9% | 35.6 | 40.0% | 96.0% | 61.5 | 62.7% | 84.4% |
| 64.1 | 68.0% | 96.9% | 36.6 | 38.0% | 96.0% | 62.3 | 62.7% | 87.5% |
| 64.3 | 66.7% | 96.9% | 38.7 | 34.0% | 96.0% | 62.8 | 61.3% | 87.5% |
| 64.9 | 65.3% | 96.9% | 40.1 | 32.0% | 96.0% | 63.0 | 60.0% | 87.5% |
| 65.3 | 64.0% | 96.9% | 41.8 | 30.0% | 96.0% | 63.7 | 58.7% | 87.5% |
| 65.6 | 62.7% | 96.9% | 44.1 | 28.0% | 96.0% | 64.7 | 57.3% | 87.5% |
| 65.7 | 61.3% | 96.9% | 45.8 | 26.0% | 96.0% | 65.2 | 56.0% | 87.5% |
| 65.8 | 60.0% | 96.9% | 46.9 | 24.0% | 96.0% | 65.7 | 54.7% | 87.5% |
| 66.2 | 58.7% | 96.9% | 47.1 | 24.0% | 100.0% | 65.9 | 53.3% | 87.5% |
| 66.7 | 57.3% | 96.9% | 47.5 | 22.0% | 100.0% | 66.7 | 52.0% | 87.5% |
| 68.1 | 56.0% | 96.9% | 51.0 | 20.0% | 100.0% | 67.6 | 52.0% | 90.6% |
| 69.7 | 54.7% | 96.9% | 55.0 | 18.0% | 100.0% | 68.1 | 50.7% | 90.6% |
| 70.1 | 53.3% | 96.9% | 55.5 | 16.0% | 100.0% | 68.5 | 49.3% | 90.6% |
| 70.3 | 52.0% | 96.9% | 56.0 | 14.0% | 100.0% | 68.6 | 48.0% | 90.6% |
| 70.6 | 50.7% | 96.9% | 57.4 | 12.0% | 100.0% | 68.6 | 46.7% | 90.6% |
| 70.8 | 49.3% | 96.9% | 59.0 | 10.0% | 100.0% | 68.8 | 45.3% | 90.6% |
| 70.8 | 49.3% | 100.0% | 59.7 | 8.0% | 100.0% | 68.9 | 45.3% | 93.8% |
| 70.9 | 48.0% | 100.0% | 61.3 | 6.0% | 100.0% | 69.7 | 44.0% | 93.8% |
| 71.3 | 46.7% | 100.0% | 67.0 | 4.0% | 100.0% | 70.5 | 42.7% | 93.8% |
| 71.7 | 45.3% | 100.0% | 75.1 | 2.0% | 100.0% | 71.1 | 41.3% | 93.8% |
| 72.0 | 44.0% | 100.0% | 80.0 | 0.0% | 100.0% | 71.6 | 40.0% | 93.8% |
| 72.8 | 42.7% | 100.0% | | | | 71.7 | 38.7% | 93.8% |
| 73.4 | 41.3% | 100.0% | | | | 71.8 | 37.3% | 93.8% |
| 73.5 | 40.0% | 100.0% | | | | 72.0 | 36.0% | 93.8% |

(continued)

| GJA12 cut-off | GJA12 sensiti vity | GJA12 specifici ty | RGN male cut-off | RGN male sensiti vity | RGN male specificity | TCEAL7 cut-off | TCEAL7 sensitivity | TCEAL7 specifici ty |
|---|---|---|---|---|---|---|---|---|
| 73.9 | 38.7% | 100.0% | | | | 72.6 | 34.7% | 93.8% |
| 74.3 | 37.3% | 100.0% | | | | 73.3 | 34.7% | 96.9% |
| 74.5 | 36.0% | 100.0% | | | | 74.3 | 33.3% | 96.9% |
| 74.8 | 34.7% | 100.0% | | | | 74.9 | 32.0% | 96.9% |
| 74.9 | 33.3% | 100.0% | | | | 75.0 | 30.7% | 96.9% |
| 75.5 | 32.0% | 100.0% | | | | 75.1 | 29.3% | 96.9% |
| 76.2 | 30.7% | 100.0% | | | | 75.3 | 29.3% | 100.0% |
| 76.6 | 29.3% | 100.0% | | | | 75.5 | 28.0% | 100.0% |
| 76.9 | 28.0% | 100.0% | | | | 75.7 | 26.7% | 100.0% |
| 77.2 | 26.7% | 100.0% | | | | 75.8 | 25.3% | 100.0% |
| 77.6 | 25.3% | 100.0% | | | | 75.9 | 24.0% | 100.0% |
| 78.1 | 24.0% | 100.0% | | | | 75.9 | 22.7% | 100.0% |
| 78.4 | 22.7% | 100.0% | | | | 76.6 | 21.3% | 100.0% |
| 78.6 | 21.3% | 100.0% | | | | 77.3 | 20.0% | 100.0% |
| 79.0 | 20.0% | 100.0% | | | | 77.9 | 18.7% | 100.0% |
| 79.8 | 18.7% | 100.0% | | | | 78.5 | 17.3% | 100.0% |
| 80.3 | 17.3% | 100.0% | | | | 79.1 | 16.0% | 100.0% |
| 80.4 | 16.0% | 100.0% | | | | 80.2 | 14.7% | 100.0% |
| 80.5 | 14.7% | 100.0% | | | | 80.8 | 13.3% | 100.0% |
| 81.2 | 13.3% | 100.0% | | | | 80.9 | 12.0% | 100.0% |
| 82.1 | 12.0% | 100.0% | | | | 81.1 | 9.3% | 100.0% |
| 82.6 | 10.7% | 100.0% | | | | 81.5 | 8.0% | 100.0% |
| 82.9 | 9.3% | 100.0% | | | | 81.9 | 6.7% | 100.0% |
| 83.1 | 8.0% | 100.0% | | | | 82.8 | 5.3% | 100.0% |
| 83.2 | 6.7% | 100.0% | | | | 83.9 | 4.0% | 100.0% |
| 83.8 | 5.3% | 100.0% | | | | 84.8 | 2.7% | 100.0% |
| 84.8 | 4.0% | 100.0% | | | | 88.2 | 1.3% | 100.0% |
| 85.5 | 2.7% | 100.0% | | | | 91.8 | 0.0% | 100.0% |
| 86.6 | 1.3% | 100.0% | | | | | | |
| 88.5 | 0.0% | 100.0% | | | | | | |

Thus in one embodiment there is provided a method as described above wherein the methylation status is scored by determining the percentage methylation of each of said genes and comparing the values to methylation cut off percentages selected from the table(s) above
wherein a value for a gene which exceeds the methylation cut off percentage for said gene is scored as ’methylated’.

Thus in one embodiment there is provided a method as described above wherein a sensitivity is selected and the methylation status is scored by determining the percentage methylation of each of said genes and comparing the values to the corresponding methylation cut off percentages for the selected sensitivity, the values selected from the table(s) above,
wherein a value for a gene which exceeds the methylation cut off percentage for said gene is scored as ’methylated’.
Thus in one embodiment there is provided a method as described above wherein a specificity is selected and the methylation status is scored by determining the percentage methylation of each of said genes and comparing the values to the corresponding methylation cut off percentages for the selected specificity, the values selected from the table(s) above,
wherein a value for a gene which exceeds the methylation cut off percentage for said gene is scored as ’methylated’.

Thus in one embodiment there is provided a method as described above wherein a specificity and sensitivity is selected and the methylation status is scored by determining the percentage methylation of each of said genes and comparing the values to the corresponding methylation cut off percentages for the selected specificity and sensitivity, the values selected from the table(s) above,
wherein a value for a gene which exceeds the methylation cut off percentage for said gene is scored as 'methylated'.

**Reference Standard**

The reference standard typically refers to a sample from a healthy individual i.e. one who does not have EAC. The reference standard may be from a healthy individual who has BE but does not have HGD/EAC, most suitably does not have EAC.
Moreover, controls may be chosen with greater precision depending on which marker is being considered. For example if considering AOL then it may be advantageous to choose a control of BE without dysplasia or EAC. For example if ploidy is being considered then it may be advantageous to choose a control of any normal tissue (normal squamous oesophagus for example).

The reference standard can an actual sample analysed in parallel. Alternatively the reference standard can be one or more values previously derived from a comparative sample e.g. a sample from a healthy subject. In such embodiments a mere numeric comparison may be made by comparing the value determined for the sample from the subject to the numeric value of a previously analysed reference sample. The advantage of this is not having to duplicate the analysis by determining concentrations in individual reference samples in parallel each time a sample from a subject is analysed.

Suitably the reference standard is matched to the subject being analysed e.g. by gender e.g. by age e.g. by ethnic background or other such criteria which are well known in the art. The reference standard may be a number such as an absolute concentration or percentage methylation value drawn up by one or more previous studies.

Reference standards may suitably be matched to specific patient sub-groups e.g. elderly subjects, or those with a previous relevant history such as acid reflux or BE.

Suitably the reference standard is matched to the sample type being analysed. For example the concentration of the biomarker polypeptide(s) or nucleic acid(s) being assayed may vary depending on the type or nature of the sample. It will be immediately apparent to the skilled worker that the concentration value(s) for the reference standard should be for the same or a comparable sample to that being tested in the method(s) of the invention. For example, if the sample being assayed is from the Barrett's segment then the reference standard value should be for Barrett's segment to ensure that it is capable of meaningful cross-comparison. Suitably the sample type for the reference standard and the sample type for the subject of interest are the same.

Table 1: Trends observed from the array analysis (EAC vs. BE). The number of female samples was too low for anything to have revealed statistical significance.

Table 1:

| Trends | | Probes | % probes | Genes | % genes | Probes within CpG islands | Probes outside of CpG Islands |
|---|---|---|---|---|---|---|---|
| **All genes** | Hypermethylation | 1952 | 7.1 | 1764 | 12.18 | 1389 | 563 |
| | Hypomethylation | 1740 | 6.3 | 1590 | 10.98 | 1114 | 626 |
| | Total | 3692 | 13.4 | 3354 | 23.17 | 2503 | 1189 |
| **Imprinted genes** | Hypermethylation | 33 | 8.5 | 17 | 33.33 | 29 | 4 |
| | Hypomethylation | 27 | 6.9 | 18 | 35.29 | 24 | 3 |
| | Total | 60 | 15.4 | 35 | 68.62 | 53 | 7 |
| **X-chromosome genes (males only)** | Hypermethylation | 24 | 2.2 | 22 | 3.66 | 20 | 4 |
| | Hypomethylation | 24 | 2.2 | 22 | 3.66 | 12 | 12 |
| | Total | 48 | 4.4 | 44 | 7.33 | 32 | 16 |

**Advantages**

Jin *et al* disclose a validation study of methylation biomarkers. Jin *et al*'s study was a candidate study. This means that genes already thought to be connected with Barrett's esophagus/neoplastic progression were studied for their methylation status. By contrast, the present disclosure undertook a prospective study. The present disclosure looked across the whole genome. The disclosure was trying to find the very best biomarkers available. The study carried out herein is unbiased. This study sought to find the very best biomarkers free of any history or prejudice present in the art.

In Jin *et al*, comparisons are repeatedly made to normal tissue, such as normal esophagal epithelial tissue. Normal esophagal epithelial tissue is a squamous epithelium. Jin *et al* consistently compared this squamous epithelium with BE, with dysplastic cells, and with EAC. By contrast, the present inventors advantageously chose a different comparator. In selecting their markers, the inventors compared dysplastic cells with Barrett's esophagus, or EAC with Barrett's esophagus. The etiology of dysplasia/EAC is that it arises from Barrett's esophagus, such as the Barrett's segment itself. Therefore, the inventors have the insight that the most relevant cells for comparison are cells from Barrett's esophagus. It is the difference between those cells and the dysplastic/EAC cells which would allow progression to be predicted/identified. Thus in one aspect the invention relates to a method of selecting a marker useful in predicting presence of or progression to dysplasia/EAC, comprising comparing markers in dysplastic cells with Barrett's esophagus, or in EAC with Barrett's esophagus, and selecting those which display differences between those cell types.

Moreover, the disclosure goes on to teach that duodenum is an excellent control tissue. This is because duodenum is a normal intestinal tissue closely related to the cells in a Barrett's esophagus segment. The cells in both these settings (i.e. Barrett's cells in a Barrett's segment and duodenum) are columnar epithelium. This is a very different tissue organisation to squamous epithelium. Therefore, by comparing possibly dysplastic or cancerous cells with Barrett's esophagus cells or with duodenal cells, a more accurate biomarker may be selected. Thus in one aspect the disclosure relates to a method of selecting a marker useful in predicting presence of or progression to dysplasia/EAC, comprising comparing markers in dysplastic cells with duodenum, or in EAC with duodenum, and selecting those which display differences between those cell types.

Biomarkers selected according to the present disclosure have the advantage of showing a difference between a more clinically relevant tissue and the lesion compared to prior art techniques which compare squamous epithelium with the lesion.

A summary of key advantages of the disclosure compared with certain publications is presented to aid understanding of the benefits of the disclosure.

| Topic | Present Invention | Jin et al. 2009 | Kaz et al. 2011 | Comments & particular advantages of the invention |
|---|---|---|---|---|
| Study Design | Methylation array discovery + internal validation + retrospective external validation + prospective external validation | Validation of 8 genes reported by different papers | Methylation array discovery | We describe the most comprehensive design |
| Gene coverage | 27,578 individual CpG loci spanning 14,475 genes and 110 miRNA promoters | The 8 targeted genes were identified from a pool of 20 genes (3 from 10-gene pool, 5 from another 10-gene pool) | 1,505 CpG sites within 807 genes | We describe much larger coverage than the other two |
| Sample size | Discovery: 22 BE; 24 EAC; Internal validation: 22 BE; 24 EAC; Retrospective: 60 BE; 36 dysplasia; 90 EAC; Prospective: a cohort of 98 paitents | 50 progressors; 145 non-porgressors | 29 SQ; 29 BE; 8HFD; 30 EAC | We describe the largest sample size |

(continued)

| Topic | Present Invention | Jin et al. 2009 | Kaz et al. 2011 | Comments & particular advantages of the invention |
|---|---|---|---|---|
| Outcome of interest | Prevalence of dysplasia | Progression to HGD/EAC | Prevalence of EAC | Although Jin et al tried to predict the progression risk, the majority of |
| | | | | their porgressors (72%) progressed 0-2 years after index biopsy, which, strictly speaking, was also detecting prevalent HGD/EAC |
| Biomarker selection | Stringent selection criteria: Signal-to-noise ratio and Wilcoxon test adjusted for multiple comparison; | Taking from previous publications | t-tests adjusted for multiple comparison | |
| Results | 6 out of 7 top genes were validated in the internal cohort; all the 6 validated in the retrospective external cohort; the top 4 of the 6 genes have good risk prediction ability in the prospective external cohort | 3 out of 8 genes were validated; the 8 gene as a panel had good accuracy | 17 genes were differently methylated between BE and EAC at adjusted significance level of 0.001. No validation available | We describe the most reliable results across different study populations and the selection of our panel was more reasonable. |
| Accuracy of the signature | AUC in the external validated cohort: 0.988 | AUC combined model (all progressors): 0.840 and 0.732 before and after correcting for overfitting respectively | N/a | We describe the best accuracy. |
| Output | Simple sum of methylation values; or count of the number of methlyated gene | a regression model with different weight on 8 genes | N/a | Our results are simpler |
| Summary | The signature was generated based on several validations and evidence is concrete | Good accuracy to predict "progression risk", but the selection of 8 gene signature needs further discussion as only 3 of them were individually validated. More validation cohorts are needed to validate the signature model. Model needs to be simplified before practical use | Simply a discovery study, long way to go before clinical usage. | |

## Further Applications

In so far as the embodiments of the disclosure described above are implemented, at least in part, using software-controlled data processing apparatus, it will be appreciated that a computer program providing such software control, and a storage medium by which such a computer program is stored, are envisaged as aspects of the present disclosure. Clearly in several of the methods or processes of the disclosure, one step (typically step (a)) comprises providing an esophagal sample from the subject - clearly that step would not typically be performed using software-controlled data processing apparatus; suitably that step is manually executed, or omitted, in embodiments implemented using software-controlled data processing apparatus.

In another aspect, the disclosure relates to a method for aiding assessment of the likelihood of dysplasia or esophageal adenocarcinoma being present in a subject, comprising carrying out the method steps as described above, wherein if 2 or more of said genes are methylated then increased likelihood of dysplasia or esophageal adenocarcinoma being present is determined.

In another aspect, the disclosure relates to a method for predicting the presence of, or the likelihood of presence of, dysplasia or esophageal adenocarcinoma in a subject, comprising carrying out the method steps as described above, wherein if 2 or more of said genes are methylated then presence of, or increased likelihood of presence of dysplasia or esophageal adenocarcinoma is predicted.

In another aspect, the disclosure relates to a method for determining a probability of, or determining a risk of, dysplasia or esophageal adenocarcinoma being present in a subject, comprising carrying out the method steps as described above, wherein if 2 or more of said genes are methylated then an increased probability of, or increased risk of, dysplasia or esophageal adenocarcinoma being present is determined.

In another aspect, the disclosure relates to a method of assessing a subject for presence of dysplasia or esophageal adenocarcinoma, comprising carrying out the method steps as described above, wherein if 2 or more of said genes are methylated then increased likelihood of presence of dysplasia or esophageal adenocarcinoma is determined.

In another aspect, the disclosure relates to a method for aiding assessment of the likelihood of dysplasia or esophageal adenocarcinoma being present in a subject, the method comprising

(a) providing an oesophagal sample from said subject
(b) determining the methylation status of

(i)*SLC22A18*,
(ii)*PIGR*,
(iii)*GJA12* and
(iv)*RIN2*

in said sample
wherein if 2 or more of said genes are methylated then an increased likelihood of presence of dysplasia or esophageal is determined.

Suitably the dysplasia is high grade dysplasia (HGD).

In another aspect, the disclosure relates to a method of assessing the risk for a particular subject comprising performing the method as described above, wherein if 0 or 1 of said genes are methylated then low risk is determined, and if 2 of said genes are methylated then intermediate risk is determined, if 2 or more of said genes are methylated then high risk is determined.

Suitably methylation status is determined by pyrosequencing.

**Brief Description of the Figures**

Figure 1: GSEA generated heat maps for the top 50 probes showing greatest differential methylation between BE and EAC (red color = high methylation, blue color = low methylation). a - all probes (22BE vs. 24EAC), b - imprinted genes probes (22BE vs. 24 EAC), c - X-chromosome probes (15BE vs. 20EAC, males only), d -X-chromosome probes (7BE vs. 4EAC, females only).

Figure 2a: Genes selected from the array analysis showing the greatest difference in methylation between BE and EAC. Beta values from the array are plotted on the x-axis against the gene name and tissue type on y-axis. 2b: For genes on the X-chromosome, analyses were separated on the basis of gender to cater for the effects of X-inactivation in females. Since *RGN* lies on the region of X-chromosome that is inactivated, males and females have different levels of methylation. Females have higher methylation in both tissues (BE and EAC) compared to males. *TCEAL7* does not appear to be affected by X-inactivation and males and females have similar levels of methylation in both BE and EAC. 2c: Methylation levels for *RGN* and *TCEAL7* in the normal esophageal epithelium in males and females using pyrosequencing.

Figure 3: Internal validation. Beta values from the Illumina Infinium array (y-axis) are plotted against the % methylation from pyrosequencing (x-axis) (N=12).

Figure 4: Retrospective external validation. N(BE) = 60, N(BED) = 36, N(EAC) = 90 for *SLC22A18*, *GJA12* and *RIN2*. N(BE) = 30, N(BED) = 6, N(EAC) = 70 for *PIGR* and *TCEAL7.* N(BE) = 45, N(BED) = 30, N(EAC) = 60 for *RGN* (Males only). Middle line = median, box = 25-75 percentile, whiskers = 10-90 percentile. *=p<0.01, **=p<0.001, ***=p<0.0001 using ANOVA.

Figure 5: ROC curves for all six targets. N(BE) = 32 vs. N(BED)+ N(EAC) = 73. For *RGN* (Males only) N(BE) = 25 vs. N(BED)+N(EAC) = 51.

Figure 6a: The four gene risk score (*SLC22A18* + *PIGR* + *GJA12* + *RIN2*) had the best AUC of 0.988 (P<0.01). 6b: Graphical representation of percentage of patients falling into each group. The probability of HDG/early EAC increases with an increase in the number of positive biomarkers.

Figure 7: Supplementary Figure 1: Internal validation for *ATP2B4*. Beta values from the Illumina Infinium array (y-axis) are plotted against the % methylation from pyrosequencing (x-axis) (N=12).

Figure 8: Supplementary Figure 2: Box plots showing no significant change in RGN methylation levels in female patient samples. N(BE) = 13, N(BED) = 6, N(EAC) = 23.

Figure 9: Supplementary Table 1: Patient demographics for the methylation arrays.

Figure 10: Supplementary Table 2: Patient demographics for retrospective external validation.

Figure 11: Supplementary Table 3: Patient demographics for prospective validation.

Figure 12: Supplementary Table 4: List of the top 30 hypermethylated genes (Genes selected for validation are marked by an asterisk).

Figure 13: Supplementary Table 5: Primer sequences and genomic co-ordinates for pyrosequencing assays.

Figure 14: Supplementary Table 6: Primer sequences for pyrosequencing controls.

Figure 15: Supplementary Table 7: Methylation cut-offs selected for maximum sensitivity and specificity.

## Examples

### Methods:

27K methylation arrays were used to find genes best able to differentiate between 22 BE and 24 esophageal adenocarcinoma (EAC) samples. These were validated using pyrosequencing on a retrospective cohort (60 BE, 36 dysplastic and 90 EAC) and then in a prospective multicenter study (100 BE patients, including 21 dysplastic and 5 early EAC) designed to utilize biomarkers to stratify patients according to their dysplasia/EAC status.

### Results:

23% of all genes on the array, including 7% of X-linked and 69% of imprinted genes, demonstrated statistically significant changes in methylation in EAC vs. BE (Wilcoxon P<0.05). 6/7 selected candidate genes were successfully internally (Pearson's P<0.01) and externally validated (ANOVA P<0.001). Four genes (*SLC22A18*, *PIGR, GJA12* and *RIN2*) were found to have the greatest area under curve (0.988) to distinguish between BE and dysplasia/EAC. This methylation panel was able to stratify patients from the prospective cohort into three risk groups based on the number of genes methylated (low risk: <2 genes, intermediate: 2 and high: >2).

**Conclusion:**

Widespread DNA methylation changes were observed in Barrett's carcinogenesis including ≈70% of known imprinted genes. A four gene methylation panel stratified BE patients into three risk groups with potential clinical utility.

**Materials and Methods:**

Patient samples:

For the retrospective studies (methylation arrays and external validation) all patient samples (H&E slides, endoscopic biopsies and surgical resection specimens), were obtained from patients who had attended Cambridge University Hospitals NHS Trust and provided individual informed consent (ethics: 04/Q2006/28, 09/H0308/118). For the prospective study patients with BE undergoing surveillance or tertiary referral for further evaluation of HGD or early EAC were recruited after obtaining informed consent from Cambridge University Hospitals NHS Trust, Queens University Hospital Nottingham and Amsterdam Medical Centre (ethics: 10/H0305/52). Pathology was verified for all cases according to the Royal College of Pathologists UK guidelines by an experienced upper GI pathologist (Dr Maria O'Donovan) and for dysplasia and EAC a minimum of two experienced pathologists reviewed the cases (referring hospital + Dr Maria O'Donovan). All BE samples were confirmed to have intestinal metaplasia and all EACs for a cellularity of ≥70%. Patient demographics are available in Supplementary Tables 1, 2 and 3.

DNA extraction and bi-sulfite conversion:

For the methylation arrays, high molecular weight DNA was isolated from fresh frozen tissue using standard proteinase-K phenol/chloroform extraction. Samples with $A_{260/280}$ of <1.8 and a fragment size of <2kb were discarded. Volume corresponding to 1μg of DNA was measured using Quant-iT™ PicoGreen® dsDNA kit (Invitrogen Ltd, UK) according to the manufacturer's instructions. Bi-sulfite modification was done using EZ DNA Methylation-Gold™ Kit (Zymo Research Corporation, USA).

DNA extraction for pyrosequencing assays was also carried out using the above mentioned protocol. DNA extraction from formalin fixed paraffin embedded (FFPE) tissues was carried out using QIAamp DNA Micro Kit (Qiagen, UK) using the manufacturer's instructions. 1μg of DNA was bi-sulfite modified and eluted in 30 μl of elution buffer.

Illumina Infinium assay:

The Infinium assay (Illumina, UK) was run using the automated protocol from Cambridge Genomic Services. The samples were denatured prior to whole genome amplification (WGA) using 0.1 N NaOH. Multi-sample amplification master mix (MSM) was then added to the DNA samples and incubated at 37°C for 20 hours. The amplified DNA was fragmented by vortexing, precipitated using isopropanol and dispensed onto the BeadChips which were incubated at 48°C for 20 hours in hybridization buffer to allow for the DNA to hybridize. Unhybridized DNA was washed off and single-base extension was carried out with extended primers and labeled nucleotides using the TECAN Freedom Evo liquid handling robot. The BeadArray Reader (Illumina) was used to read the signal and output files were generated using GenomeStudio Software (Illumina).

Array data analysis and selecting targets:

a. Signal-to-noise ratio ranking: BE and EAC samples were separated into two groups and ranking of genes was done using the 'Signal2Noise' metric (GSEA software, Broad Institute, USA). Signal2Noise uses the difference of means scaled by the standard deviation.

$$(\mu A - \mu B)/(\sigma A + \sigma B)$$

where μ is the mean and σ is the standard deviation. The larger the signal-to-noise ratio, the larger the difference of means (scaled by standard deviation); hence more distinct methylation is seen for each phenotype and more the gene acts as a 'class marker'. Imprinted genes and those on the X-chromosome were analyzed separately. The final list of genes can be obtained from Supplementary Table 4.

b. Wilcoxon tests: As a further check to test for differential methylation, a two-sided Wilcoxon test was performed for each probe on the array. Variance of probes with low or high methylation is in general lower than variance of probes with medium methylation[22]. So tests for differential methylation tend to preferentially select probes whose values are confined to the extremes of the scale. To reduce this effect we performed a Gaussian normalization prior to the Wilcoxon tests to reduce heteroscedasticity. The values' ranks, normalized between 0 and 1, were taken to be probabilities from a Gaussian distribution and transformed to variables using the distribution's quantile function. The P-values were adjusted for multiple testing using the false discovery rate method of Benjamini & Hochberg[23]. We were interested in probes that had both statistically significant and large absolute differences in methylation. Therefore, for each probe we also calculated the difference between the median of the methylation values in the two phenotypes. A probe's rank in the ordered list of Wilcoxon P-values and its rank in the ordered list of absolute difference in medians were averaged. The probes were arranged in descending order of this average.

The purpose of using two different tests to look for targets was to avoid false positives and to ensure that the selected targets not only have a statistically significant but a large absolute difference in methylation that was reproducible using pyrosequencing which has an error margin of ±5%. The targets appearing high up in both these analyses were then selected for validation.

Genes were selected for validation based on the following criteria: present in both of the lists, biological importance in EAC and/or other cancers, proximity to the promoter and relatively low density of CpGs in the vicinity so that it would be possible to design robust pyrosequencing assays (Figure 2a and 2b, Supplementary Table 4).

Pyrosequencing assays:

Pyrosequencing assays were designed using PSQ Assay Design Software (version 1.0.6, Biotage, Sweden) (Supplementary Table 5). Genomic DNA sequences were obtained from NCBI map viewer (build 36). All PCR reactions were carried out in volumes of 25μl using IMMOLASE™ DNA Polymerase (Bioline, UK). 0.75μl of bi-sulfite converted DNA was used as a template for each reaction. 20μl of each PCR reaction was mixed with 60μl of bead mix composed of 3μl streptavidin-coated beads solution (GE Healthcare, UK), 20ul nuclease free water and 37μl PyroMark binding buffer (Qiagen) in a 96-well plate and left on a shaking platform for 10 min. The pyrosequencing reaction plate was prepared by adding 1.5μl of 10μM sequencing primer and 43.5μl of PyroMark Annealing Buffer (Qiagen) into each of the wells. The pyrosequencing vacuum machine (Biotage) was used to wash and denature the DNA bound to streptavidin-coated beads before being released into the pyrosequencing reaction plate. The plate was heated to 80°C for 3 min and then cooled down to room temperature to allow the sequencing primer to anneal onto the single-stranded DNA and the sequencing reaction was carried out according to the manufacturers' protocol.

0%, 50% and 100% methylated controls were prepared for all the assays and used with every run. DNA synthesized by PCR was used for this. Primers were designed using the NCBI Primer Designing Tool (http://www.ncbi.nlm.gov/tools/primer-blast/index.cgi) in order to amplify a region greater than but containing the sequence to be analyzed by pyrosequencing (Supplementary Table 6). Genomic DNA isolated from normal squamous esophagus was used as a template. All PCRs were performed in 50μl duplicates. One reaction was used for in-vitro methylation. Briefly 40μl of the PCR reaction was mixed with 5μl of 10×NEBuffer2, 2.5μl of 3.2 mM S-adenosylmethionine (SAM), 4U (1μl) of CpG Methyltransferase (M.SssI) (NEB, UK) and incubated for 2 hours at 37°C. After 2 hours another 0.5μl of 3.2mM SAM, 2U (0.5μl) of M.SssI and 0.5μl of water were added and incubated overnight at 37°C. Both reactions (in-vitro methylated and unmethylated) were then purified using QIAquick PCR purification Kit (Qiagen). These were then bisulfite converted as mentioned before and mixed to generate a 50% methylated control along with 0% and 100% methylated controls.

**Example 1**

Widespread changes in DNA methylation were observed between BE and EAC:

Illumina HumanMethylation27 BeadChips were used to assess and compare methylation levels of 27,578 individual CpG loci spanning 14,475 genes and 110 miRNA promoters in 22 BE and 24 EAC samples (GEO accession no: GSE32925). Signal-to-noise ratio and two-sided Wilcoxon tests were used to rank genes showing the greatest difference in methylation (both hypermethylation and hypomethylation) between the BE and EAC, and from this a 'class marker' gene set was identified that was able to clearly distinguish between the two phenotypes (Figure 1). 23% of all the genes present on the array showed a statistically significant difference in methylation (Wilcoxon P<0.05). On the whole hypermethylation was observed to be slightly more prevalent (1,764/14,475 - 12.18%) as compared to hypomethylation (1,590/14,475- 10.98%) in EAC vs. BE (Wilcoxon P<0.05). Out of the 51 imprinted genes present on the array (list obtained from www.geneimprint.com) 17 (33.33%) showed hypermethylation and 18 (35.29%) hypomethylation in EAC vs. BE (Wilcoxon P<0.05) (which comes to a total of 68.62% of all the imprinted genes present on the array).

Separate analyses were done for males and females for genes on the X-chromosome to cater for the effects of X-inactivation in females. Genes on the X-chromosome showed similar levels of hyper and hypomethylation in EAC compared to BE (22 genes each hyper and hypomethylated out of a total 600, Wilcoxon P<0.05). Most methylation changes were confined to within known CpG islands. Detailed results can be seen in Table 1.

<u>Targets were identified to have a statistically significant and large absolute difference in methylation between BE and EAC:</u>

To ensure that the selected targets for validation would have a statistically significant and large absolute difference in methylation and hence be suitable as biomarkers, the results of signal-to-noise ratio ranking were compared to the results of the Wilcoxon tests. The top seven genes present in both the lists fulfilling the aforementioned selection criteria (see methods) were selected for validation (Figure 2a). For *RGN* which is an X-inactivated gene (p11.3-Xp11.23) it was observed that methylation levels were different in males compared to females in normal tissues (normal squamous esophageal epithelium). Therefore, separate analyses were done for both the genders for *RGN* in the pathological external validation samples. *TCEAL7* on the other hand, also on the X-chromosome, did not appear to be affected by DNA methylation associated X-inactivation and therefore the analysis for males and females were combined in all subsequent experiments (Figure 2b and 2c).

These seven genes were first internally validated using pyrosequencing assays on the same samples that were run on the methylation arrays. The assays were designed to analyze the same DNA sequence which was probed by the arrays. Pearson's correlation was used to assess whether the results from pyrosequencing matched with the results from the arrays (Fig. 3). Six out of seven genes successfully validated which were *SLC22A18* (tumor suppressing subtransferable candidate 5, a paternally imprinted gene) (P<0.0001, coefficient=0.9), *PIGR* (polymeric immunoglobulin receptor) (P<0.0001, coefficient=0.9), *GJA12* (gap junction protein, gamma 2) (P<0.0001, coefficient= 0.9), *RIN2* (Ras and Rab interactor 2) (P<0.01, coefficient= 0.7), *RGN* (senescence marker protein-30, X-linked gene) (P<0.0001, coefficient= 0.9) and *TCEAL7* (transcription elongation factor A - like 7, X-linked gene) (P<0.0001, coefficient=0.9). *ATP2B4* however failed to validate (P=0.6, coefficient=0.1) as shown in Supplementary Figure 1.

<u>Retrospective external validation of selected targets using pyrosequencing showed a consistent statistically significant increase in DNA methylation through the metaplasia-dysplasia-adenocarcinoma sequence:</u>

External validation by pyrosequencing was carried out on an independent set of 60 BE, 36 BE with dysplasia and 90 EAC samples (Figure 4). All of these cases had the histopathological diagnosis confirmed on the actual biopsy used for analysis. This validation set also enabled an assessment to be made of when in the disease pathogenesis the methylation changes occurred. A statistically significant increase in methylation was observed for all the selected biomarker genes in EAC and/or dysplastic BE compared to non-dysplastic BE (ANOVA P<0.001). For *SLC22A18, PIGR, TCEAL7* and *RIN2* genes it was a gradual increase, whereas for *RGN* the biggest change in methylation occurred at the onset of dysplasia and for *GJA12* this occurred between dysplasia and EAC.

<u>Methylation can distinguish non-dysplastic BE from dysplastic BE and EAC:</u>

Since an increase in DNA methylation was observed in EAC and dysplastic BE compared to non-dysplastic BE, ROC curves were used to detect the power of the 6 genes individually and then in combination to differentiate between dysplastic BE/EAC and non-dysplastic BE (Figure 5, Supplementary Table 7). Individually *GJA12* (AUC=0.973) was best able to distinguish between dysplasia/EAC and non-dysplastic BE followed by *PIGR* (AUC=0.963), *SW22A18* (AUC=0.954), *RIN2* (0.922), *RGN* (AUC=0.865) but only in males and lastly *TCEAL* (AUC=0.788). The greatest AUC of 0.988 (P<0.01) was obtained using the four gene combination (*SLC22A18* + *PIGR* + *GJA12* + *RIN2*) which had a sensitivity of 94% and a specificity of 97% (Figure 6a).

<u>DNA methylation can stratify BE patients into three risk groups; low, intermediate and high risk:</u>

The methylation cut-offs selected for the four genes using ROC curves (*SLC22A18, PIGR, GJA12, RIN2*) were then tested on a prospective cohort of 100 patients (including 21 dysplastic and 5 EAC cases) undergoing BE surveillance endoscopy in three tertiary referral centers to enrich for dysplasia and EAC. Random quadrantic biopsies every 2 cm were taken according British Society of Gastroenterology guidelines (<u>http://www.bsg.org.uk/pdf word docs/Barretts Oes.pdf</u>) along with 3 extra biopsies for DNA methylation taken randomly from within the BE segment. For the analysis, the biopsy with the highest methylation value per gene was selected taking advantage of the likely molecular field effect. A patient was categorized according to their highest histopathological diagnosis (LGD<HGD<EAC) on any surveillance biopsy taken at that endoscopy. The data demonstrated that the risk of both dysplasia and EAC increased with the number of genes methylated (Figure 6b). 11.1% of the cases in the 0-1 gene methylated group were dysplastic (low grade dysplasia only). In the group with 2 genes methylated the proportion of dysplastic cases increased to 22.2% but there were no EAC cases. In the group with 3-4 genes methylated 23.6% of cases had HGD and 9.05% had EAC (combined cases of dysplasia and EAC: 32.7%). It should be noted that these data were derived from minimal sampling (3 biopsies for methylation study regardless of segment length) compared with the quadrantic biopsies taken every 2 cm to determine the histopathological diagnosis. The clinical variables such as age and sex did not alter the risk for prevalent dysplasia and EAC observed. The mean segment length in non-dysplastic BE was observed to be 7.3 cm (range 2-14 cm) and 7.1 cm (range 3-16 cm) in cases with dysplasia/EAC (MWU P=0.6).

**References**

1. Hvid-Jensen F, Pedersen L, Drewes AM, Sørensen HT, Funch-Jensen P. Incidence of Adenocarcinoma among Patients with Barrett's Esophagus. New England Journal of Medicine 2011;365:1375-1383.
2. Pohl H, Sirovich B, Welch HG. Esophageal Adenocarcinoma Incidence: Are We Reaching the Peak? Cancer Epidemiology Biomarkers & Prevention 2010;19:1468-1470.
3. Crane SJ, Locke GR, Harmsen WS, Zinsmeister AR, Romero Y, Talley NJ. Survival Trends in Patients With Gastric and Esophageal Adenocarcinomas: A Population-Based Study. Mayo Clinic Proceedings 2008;83:1087-1094.
4. Spechler SJ, Sharma P, Souza RF, Inadomi JM, Shaheen NJ. American Gastroenterological Association Technical Review on the Management of Barrett's Esophagus. Gastroenterology 2011;140:e18-e52.
5. DeMeester S. Evaluation and Treatment of Superficial Esophageal Cancer. Journal of Gastrointestinal Surgery 2010:14:94-100.
6. Yousef F, Cardwell C, Cantwell MM, Galway K, Johnston BT, Murray L. The Incidence of Esophageal Cancer and High-Grade Dysplasia in Barrett's Esophagus: A Systematic Review and Meta-Analysis. American Journal of Epidemiology 2008;168:237-249.
7. Kahrilas PJ. The Problems with Surveillance of Barrett's Esophagus. New England Journal of Medicine 2011;365:1437-1438.
8. Desai TK, Krishnan K, Samala N, Singh J, Cluley J, Perla S, Howden CW. The incidence of oesophageal adenocarcinoma in non-dysplastic Barrett's oesophagus: a meta-analysis. Gut 2011.
9. Goldblum JR. Controversies in the Diagnosis of Barrett Esophagus and Barrett-Related Dysplasia: One Pathologist's Perspective. Archives of Pathology & Laboratory Medicine 2010;134:1479-1484.
10. Downs-Kelly E, Mendelin JE, Bennett AE, Castilla E, Henricks WH, Schoenfield L, Skacel M, Yerian L, Rice TW, Rybicki LA, Bronner MP, Goldblum JR. Poor Interobserver Agreement in the Distinction of High-Grade Dysplasia and Adenocarcinoma in Pretreatment Barrett's Esophagus Biopsies. Am J Gastroenterol 2008;103:2333-2340.
11. Curvers WL, ten Kate FJ, Krishnadath KK, Visser M, Elzer B, Baak LC, Bohmer C, Mallant-Hent RC, van Oijen A, Naber AH, Scholten P, Busch OR, Blaauwgeers HG, Meijer GA, Bergman JJ. Low-grade dysplasia in Barrett's esophagus: overdiagnosed and underestimated. Am J Gastroenterol 2010;105:1523-30.
12. Wani S, Falk GW, Post J, Yerian L, Hall M, Wang A, Gupta N, Gaddam S, Singh M, Singh V, Chuang KY, Boolchand V, Gavini H, Kuczynski J, Sud P, Bansal A, Rastogi A, Mathur SC, Young P, Cash B, Goldblum J, Lieberman DA, Sampliner RE, Sharma P. Risk Factors for Progression of Low-Grade Dysplasia in Patients With Barrett's Esophagus. Gastroenterology 2011;141:1179-1186 e1.
13. Shaheen NJ, Sharma P, Overholt BF, Wolfsen HC, Sampliner RE, Wang KK, Galanko JA, Bronner MP, Goldblum JR, Bennett AE, Jobe BA, Eisen GM, Fennerty MB, Hunter JG, Fleischer DE, Sharma VK, Hawes RH, Hoffman BJ, Rothstein RI, Gordon SR, Mashimo H, Chang KJ, Muthusamy VR, Edmundowicz SA, Spechler SJ, Siddiqui AA, Souza RF, Infantolino A, Falk GW, Kimmey MB, Madanick RD, Chak A, Lightdale CJ. Radiofrequency Ablation in Barrett's Esophagus with Dysplasia. New England Journal of Medicine 2009;360:2277-2288.
14. Esteller M. Epigenetics in Cancer. New England Journal of Medicine 2008;358: 1148-1159.
15. Schulmann K, Sterian A, Berki A, Yin J, Sato F, Xu Y, Olaru A, Wang S, Mori Y, Deacu E, Hamilton J, Kan T, Krasna MJ, Beer DG, Pepe MS, Abraham JM, Feng Z, Schmiegel W, Greenwald BD, Meltzer SJ. Inactivation of p16, RUNX3, and HPP1 occurs early in Barrett's-associated neoplastic progression and predicts progression risk. Oncogene 2005;24:4138-4148.
16. Clement G, Braunschweig R, Pasquier N, Bosman FT, Benhattar J. Alterations of the Wnt signaling pathway during the neoplastic progression of Barrett's esophagus. Oncogene 2006;25:3084-3092.
17. Jin Z, Cheng Y, Olaru A, Kan T, Yang J, Paun B, Ito T, Hamilton JP, David S, Agarwal R, Selaru FM, Sato F, Abraham JM, Beer DG, Mori Y, Shimada Y, Meltzer SJ. Promoter hypermethylation of CDH13 is a common, early event in human esophageal adenocarcinogenesis and correlates with clinical risk factors. International Journal of Cancer 2008;123:2331-2336.

18. Huang Y, Peters CJ, Fitzgerald RC, Gjerset RA. Progressive silencing of p14ARF in oesophageal adenocarcinoma. Journal of Cellular and Molecular Medicine 2009;13:398-409.

19. Kuester D, El-Rifai We, Peng D, Ruemmele P, Kroeckel I, Peters B, Moskaluk CA, Stolte M, Mönkemüller K, Meyer F, Schulz H-U, Hartmann A, Roessner A, Schneider-Stock R. Silencing of MGMT expression by promoter hypermethylation in the metaplasia-dysplasia-carcinoma sequence of Barrett's esophagus. Cancer Letters 2009;275:117-126.

20. Jin Z, Cheng Y, Gu W, Zheng Y, Sato F, Mori Y, Olaru AV, Paun BC, Yang J, Kan T, Ito T, Hamilton JP, Selaru FM, Agarwal R, David S, Abraham JM, Wolfsen HC, Wallace MB, Shaheen NJ, Washington K, Wang J, Canto MI, Bhattacharyya A, Nelson MA, Wagner PD, Romero Y, Wang KK, Feng Z, Sampliner RE, Meltzer SJ. A Multicenter, Double-Blinded Validation Study of Methylation Biomarkers for Progression Prediction in Barrett's Esophagus. Cancer Research 2009;69:4112-4115.

21. Taby R, Issa J-PJ. Cancer Epigenetics. CA: A Cancer Journal for Clinicians 2010;60:376-392.

22. Laird PW. Principles and challenges of genome-wide DNA methylation analysis. Nat Rev Genet 2010;11:191-203.

23. Benjamini Y, Hochberg Y. Controlling the False Discovery Rate: A Practical and Powerful Approach to Multiple Testing. Journal of the Royal Statistical Society. Series B (Methodological) 1995;57:289-300.

24. Wang JS, Guo M, Montgomery EA, Thompson RE, Cosby H, Hicks L, Wang S, Herman JG, Canto MI. DNA Promoter Hypermethylation of p16 and APC Predicts Neoplastic Progression in Barrett's Esophagus. Am J Gastroenterol 2009;104:2153-2160.

25. Jin Z, Cheng Y, Gu W, Zheng Y, Sato F, Mori Y, Olaru AV, Paun BC, Yang J, Kan T, Ito T, Hamilton JP, Selaru FM, Agarwal R, David S, Abraham JM, Wolfsen HC, Wallace MB, Shaheen NJ, Washington K, Wang J, Canto MI, Bhattacharyya A, Nelson MA, Wagner PD, Romero Y, Wang KK, Feng Z, Sampliner RE, Meltzer SJ. A Multicenter, Double-Blinded Validation Study of Methylation Biomarkers for Progression Prediction in Barrett's Esophagus. Cancer Res 2009;69:4112-4115.

26. Jin Z, Hamilton JP, Yang J, Mori Y, Olaru A, Sato F, Ito T, Kan T, Cheng Y, Paun B, David S, Beer DG, Agarwal R, Abraham JM, Meltzer SJ. Hypermethylation of the AKAP12 Promoter is a Biomarker of Barrett's-Associated Esophageal Neoplastic Progression. Cancer Epidemiol Biomarkers Prev 2008;17:111-117.

27. Jin Z, Olaru A, Yang J, Sato F, Cheng Y, Kan T, Mori Y, Mantzur C, Paun B, Hamilton JP, Ito T, Wang S, David S, Agarwal R, Beer DG, Abraham JM, Meltzer SJ. Hypermethylation of Tachykinin-1 Is a Potential Biomarker in Human Esophageal Cancer. Clin Cancer Res 2007;13:6293-6300.

28. Tost J, Gut IG. DNA methylation analysis by pyrosequencing. Nat. Protocols 2007;2:2265-2275.

29. Alvarez H, Opalinska J, Zhou L, Sohal D, Fazzari MJ, Yu Y, Montagna C, Montgomery EA, Canto M, Dunbar KB, Wang J, Roa JC, Mo Y, Bhagat T, Ramesh KH, Cannizzaro L, Mollenhauer J, Thompson RF, Suzuki M, Meltzer S, Melnick A, Greally JM, Maitra A, Verma A. Widespread Hypomethylation Occurs Early and Synergizes with Gene Amplification during Esophageal Carcinogenesis. PLoS Genet 2011:7:e1001356.

30. Chu S-H, Feng D-F, Ma Y-B, Zhang H, Zhu Z-A, Li Z-Q, Jiang P-C. Promoter methylation and downregulation of SLC22A18 are associated with the development and progression of human glioma. Journal of Translational Medicine 2011;9:156.

31. Gallagher E, Mc Goldrick A, Chung WY, Mc Cormack O, Harrison M, Kerin M, Dervan PA, Mc Cann A. Gain of imprinting of SLC22A18 sense and antisense transcripts in human breast cancer. Genomics 2006;88:12-17.

32. Schwienbacher C, Gramantieri L, Scelfo R, Veronese A, Calin GA, Bolondi L, Croce CM, Barbanti-Brodano G, Negrini M. Gain of imprinting at chromosome 11p15: A pathogenetic mechanism identified in human hepatocarcinomas. Proceedings of the National Academy of Sciences 2000;97:5445-5449.

33. Yamaguchi M, Daimon Y. Overexpression of regucalcin suppresses cell proliferation in cloned rat hepatoma H4-II-E cells: Involvement of intracellular signaling factors and cell cycle-related genes. Journal of Cellular Biochemistry 2005;95:1169-1177.

34. Maia C, Santos C, Schmitt F, Socorro S. Regucalcin is under-expressed in human breast and prostate cancers: Effect of sex steroid hormones. Journal of Cellular Biochemistry 2009;107:667-676.

35. Kerkhof M, Van Dekken H, Steyerberg EW, Meijer GA, Mulder AH, De Bruïne A, Driessen A, Ten Kate FJ, Kusters JG, Kuipers EJ, Siersema PD, for the Csg. Grading of dysplasia in Barrett's oesophagus: substantial interobserver variation between general and gastrointestinal pathologists. Histopathology 2007;50:920-927.

36. Belshaw NJ, Elliott GO, Foxall RJ, Dainty JR, Pal N, Coupe A, Garg D, Bradburn DM, Mathers JC, Johnson IT. Profiling CpG island field methylation in both morphologically normal and neoplastic human colonic mucosa. Br J Cancer 2008;99:136-142.

37. Shen L, Kondo Y, Rosner GL, Xiao L, Hernandez NS, Vilaythong J, Houlihan PS, Krouse RS, Prasad AR, Einspahr JG, Buckmeier J, Alberts DS, Hamilton SR, Issa J-PJ. MGMT Promoter Methylation and Field Defect in Sporadic Colorectal Cancer. Journal of the National Cancer Institute 2005;97:1330-1338.

38. Leedham SJ, Preston SL, McDonald SAC, Elia G, Bhandari P, Poller D, Harrison R, Novelli MR, Jankowski JA, Wright NA. Individual crypt genetic heterogeneity and the origin of metaplastic glandular epithelium in human Barrett's oesophagus. Gut 2008;57: 1041-1048.
39. Merlo LMF, Shah NA, Li X, Blount PL, Vaughan TL, Reid BJ, Maley CC. A Comprehensive Survey of Clonal Diversity Measures in Barrett's Esophagus as Biomarkers of Progression to Esophageal Adenocarcinoma. Cancer Prevention Research 2010;3:1388-1397.
40. Reid BJ, Kostadinov R, Maley CC. New Strategies in Barrett's Esophagus: Integrating Clonal Evolutionary Theory with Clinical Management. Clinical Cancer Research 2011;17:3512-3519.

## Claims

1. An *in vitro* method for aiding assessment of the likelihood of dysplasia or esophagal adenocarcinoma being present in a subject,
wherein the sample is from a subject having Barrett's Esophagus,
the method comprising

    (a) providing an esophagal sample from said subject
    (b) determining the methylation status of

        (i) *SLC22A18,*
        (ii) *PIGR,*
        (iii) *GJA12* and
        (iv) *RIN2*

    in said sample;
    comparing the methylation values of (b) to a reference standard, wherein said reference standard is from a subject having Barrett's esophagus, but not having dysplasia or esophagal adenocarcinoma;
    wherein if 2 or more of said genes are methylated at a level higher than the reference standard then an increased likelihood of presence of dysplasia or esophagal adenocarcinoma is determined.

2. An *in vitro* method according to claim 1 wherein the method further comprises determining the methylation status of (v) TCEAL7.

3. An *in vitro* method according to claim 1 or claim 2 wherein if said subject is male, the method further comprises determining the methylation status of (vi) RGN.

4. An *in vitro* method of assessing the risk for a particular subject comprising performing the method according to any preceding claim, wherein if 0 or 1 of said genes are methylated then low risk is determined, and if 2 of said genes are methylated then intermediate risk is determined, if 3 or more of said genes are methylated then high risk is determined.

5. An *in vitro* method according to any preceding claim wherein methylation status is determined by pyrosequencing, wherein said pyrosequencing is carried out using one or more sequencing primers selected from Supplementary Table 5.

6. An *in vitro* method according to any preceding claim wherein the methylation status is scored by determining the percentage methylation of each of said genes and comparing the values to the following methylation cut off percentages:

| Gene | Methylation cut-off (%) |
|---|---|
| GJA12 | 51.74000 |
| SLC22A18 | 49.25000 |
| PIGR | 64.755000 |
| RIN2 | 37.85500 |
| *RGN* (males only) | 18.645000 |

(continued)

| Gene | Methylation cut-off (%) |
|---|---|
| TCEAL7 | 58.54000 |

wherein a value for a gene which exceeds the methylation cut off percentage for said gene is scored as 'methylated'.

7. An *in vitro* method according to any preceding claim wherein said sample comprises frozen biopsy material.

8. An *in vitro* method according to any preceding claim wherein said reference standard comprises columnar epithelium such as Barrett's esophagus or duodenum.

**Patentansprüche**

1. *In-vitro*-Verfahren zur unterstützenden Beurteilung der Wahrscheinlichkeit von Dysplasie oder Ösophagusadenokarzinom bei einem Patienten,
wobei die Probe von einem Patienten mit Barrett-Ösophagus stammt,
wobei das Verfahren umfasst

(a) Bereitstellen einer ösophagealen Probe von dem Patienten
(b) Bestimmen des Methylierungsstatus von

(i) *SLC22A18,*
(ii) *PIGR,*
(iii) *GJA12* und
(iv) *RIN2*

in der Probe;
Vergleichen der Methylierungswerte von (b) mit einem Referenzstandard, wobei der Referenzstandard von einem Patienten mit Barrett-Ösophagus, aber ohne Dysplasie oder Ösophagusadenokarzinom stammt;
wobei wenn 2 oder mehr der Gene auf einem höheren Niveau als der Referenzstandard methyliert sind, dann eine erhöhte Wahrscheinlichkeit der Anwesenheit von Dysplasie oder Ösophagusadenokarzinom bestimmt wird.

2. *In-vitro*-Verfahren nach Anspruch 1, wobei das Verfahren weiter die Bestimmung des Methylierungsstatus von (v) TCEAL7 umfasst.

3. *In-vitro*-Verfahren nach Anspruch 1 oder Anspruch 2, wobei wenn der Patient männlich ist, das Verfahren weiter die Bestimmung des Methylierungsstatus von (vi) RGN umfasst.

4. *In-vitro*-Verfahren zur Beurteilung des Risikos für einen bestimmten Patienten, umfassend die Durchführung des Verfahrens gemäß einem vorstehenden Anspruch, wobei wenn 0 oder 1 der Gene methyliert sind, dann ein geringes Risiko bestimmt wird, und wenn 2 der Gene methyliert sind, dann ein Zwischenrisiko bestimmt wird, wenn 3 oder mehr der Gene methyliert sind, dann ein hohes Risiko bestimmt wird.

5. *In-vitro*-Verfahren nach einem vorstehenden Anspruch, wobei der Methylierungsstatus durch Pyrosequenzierung bestimmt wird, wobei die Pyrosequenzierung unter Verwendung eines oder mehrerer Sequenzierprimer, ausgewählt aus der Ergänzungstabelle 5, durchgeführt wird.

6. *In-vitro*-Verfahren nach einem vorstehenden Anspruch, wobei der Methylierungsstatus durch Bestimmung der prozentualen Methylierung jedes der Gene und durch Vergleich der Werte mit den folgenden Methylierungs-Grenzwert-Prozentsätzen ermittelt wird:

| Gen | Methylierungs-Grenzwert (%) |
|---|---|
| GJA12 | 51,74000 |
| SLC22A18 | 49,25000 |

(fortgesetzt)

| Gen | Methylierungs-Grenzwert (%) |
|---|---|
| PIGR | 64,755000 |
| RIN2 | 37,85500 |
| *RGN* (nur männliche) | 18,645000 |
| TCEAL7 | 58,54000 |

wobei ein Wert für ein Gen, der den Methylierungs-Grenzwert-Prozentsatz für das Gen übersteigt, als 'methyliert' gewertet wird.

**7.** *In-vitro*-Verfahren nach einem vorstehenden Anspruch, wobei die Probe gefrorenes Biopsiematerial umfasst.

**8.** *In-vitro*-Verfahren nach einem vorstehenden Anspruch, wobei der Referenzstandard säulenförmiges Epithel, wie Barrett-Ösophagus oder Zwölffingerdarm, umfasst.

## Revendications

**1.** Procédé *in vitro* pour aider à évaluer la probabilité d'une dysplasie ou d'un adénocarcinome de l'oesophage présent chez un sujet, dans lequel l'échantillon provient d'un sujet ayant un oesophage de Barrett, le procédé comprenant :

(a) fournir un échantillon d'oesophage du sujet ;
(b) déterminer le statut de méthylation de :

(i) *SLC22A18* ;
(ii) *PIGR* ;
(iii) *GJA12* ; et
(iv) *RIN2*

dans ledit échantillon ;
comparer les valeurs de méthylation de (b) à un étalon de référence, dans lequel ledit étalon de référence provient d'un sujet ayant un oesophage de Barrett, mais n'ayant pas de dysplasie ou adénocarcinome de l'oesophage ;
dans lequel si 2 ou plus desdits gènes sont méthylés à un niveau supérieur à l'étalon de référence, alors une probabilité accrue de présence de dysplasie ou d'adénocarcinome de l'oesophage est déterminée.

**2.** Procédé *in vitro* selon la revendication 1, dans lequel le procédé comprend en outre la détermination du statut de méthylation de (v) TCEAL7.

**3.** Procédé *in vitro* selon la revendication 1 ou la revendication 2, dans lequel si ledit sujet est un homme, le procédé comprend en outre la détermination de l'état de méthylation de (vi) RGN.

**4.** Procédé *in vitro* d'évaluation du risque pour un sujet particulier comprenant la mise en oeuvre du procédé selon l'une quelconque des revendications précédentes, dans laquelle si 0 ou 1 desdits gènes sont méthylés, alors un risque faible est déterminé, et si 2 desdits gènes sont méthylés alors un risque intermédiaire est déterminé, si 3 ou plus de ces gènes sont méthylés alors un risque élevé est déterminé.

**5.** Procédé *in vitro* selon l'une quelconque des revendications précédentes, dans lequel l'état de méthylation est déterminé par pyroséquençage, dans lequel ledit pyroséquençage est effectué en utilisant une ou plusieurs amorces de séquençage choisies parmi le Tableau additionnel 5.

**6.** Procédé *in vitro* selon l'une quelconque des revendications précédentes dans lequel l'état de méthylation est noté en déterminant le pourcentage de méthylation de chacun desdits gènes et en comparant les valeurs aux pourcentages de seuil de méthylation suivants :

| Gène | Seuil de méthylation (%) |
|---|---|
| GJA12 | 51,74000 |
| SLC22A18 | 49,25000 |
| PIGR | 64,755000 |
| RIN2 | 37,85500 |
| RGN (hommes uniquement) | 18,645000 |
| TCEAL7 | 58,54000 |

dans lequel une valeur pour un gène excédant le pourcentage seuil de méthylation pour ledit gène est noté comme "méthylé".

**7.** Procédé *in vitro* selon l'une quelconque des revendications précédentes, dans lequel ledit échantillon comprend un matériau de biopsie congelé.

**8.** Procédé *in vitro* selon l'une quelconque des revendications précédentes, dans lequel ledit étalon de référence comprend un épithélium colonnaire par exemple un oesophage de Barrett ou duodénum.

Figure 1:

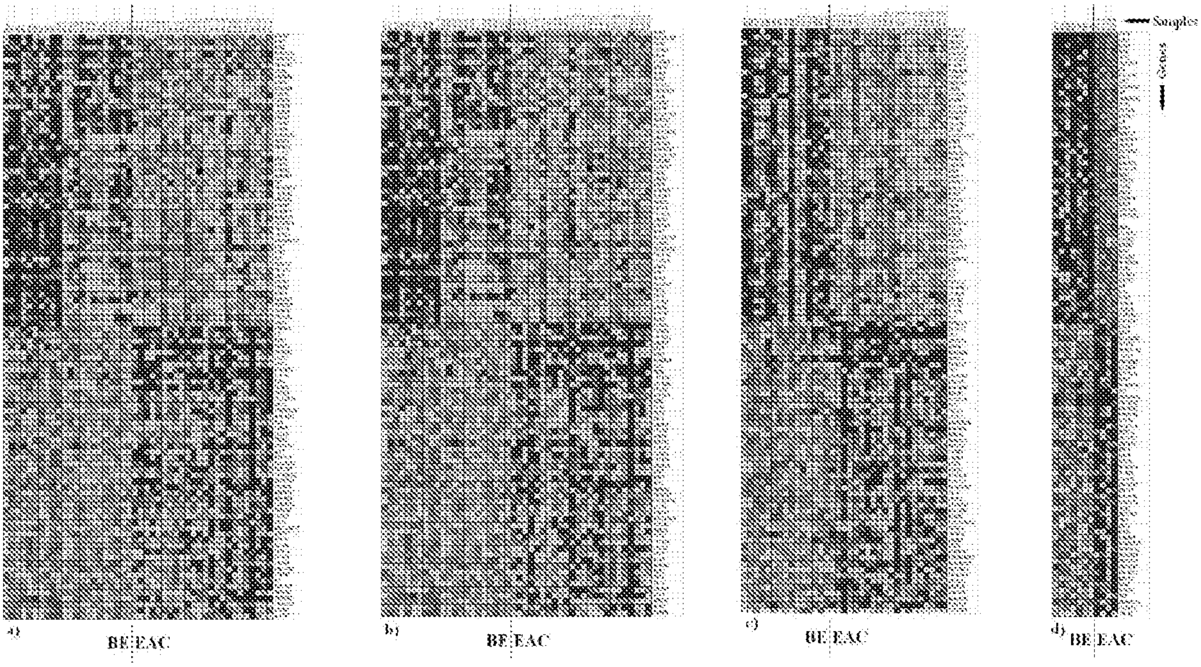


Figure 2:

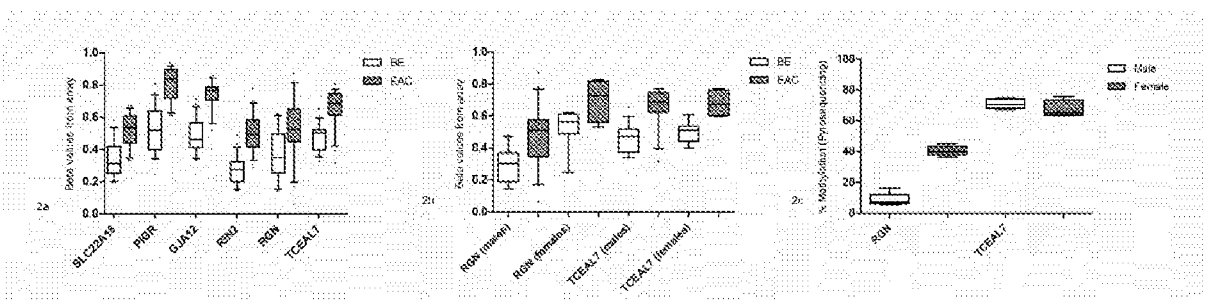

Figure 3:

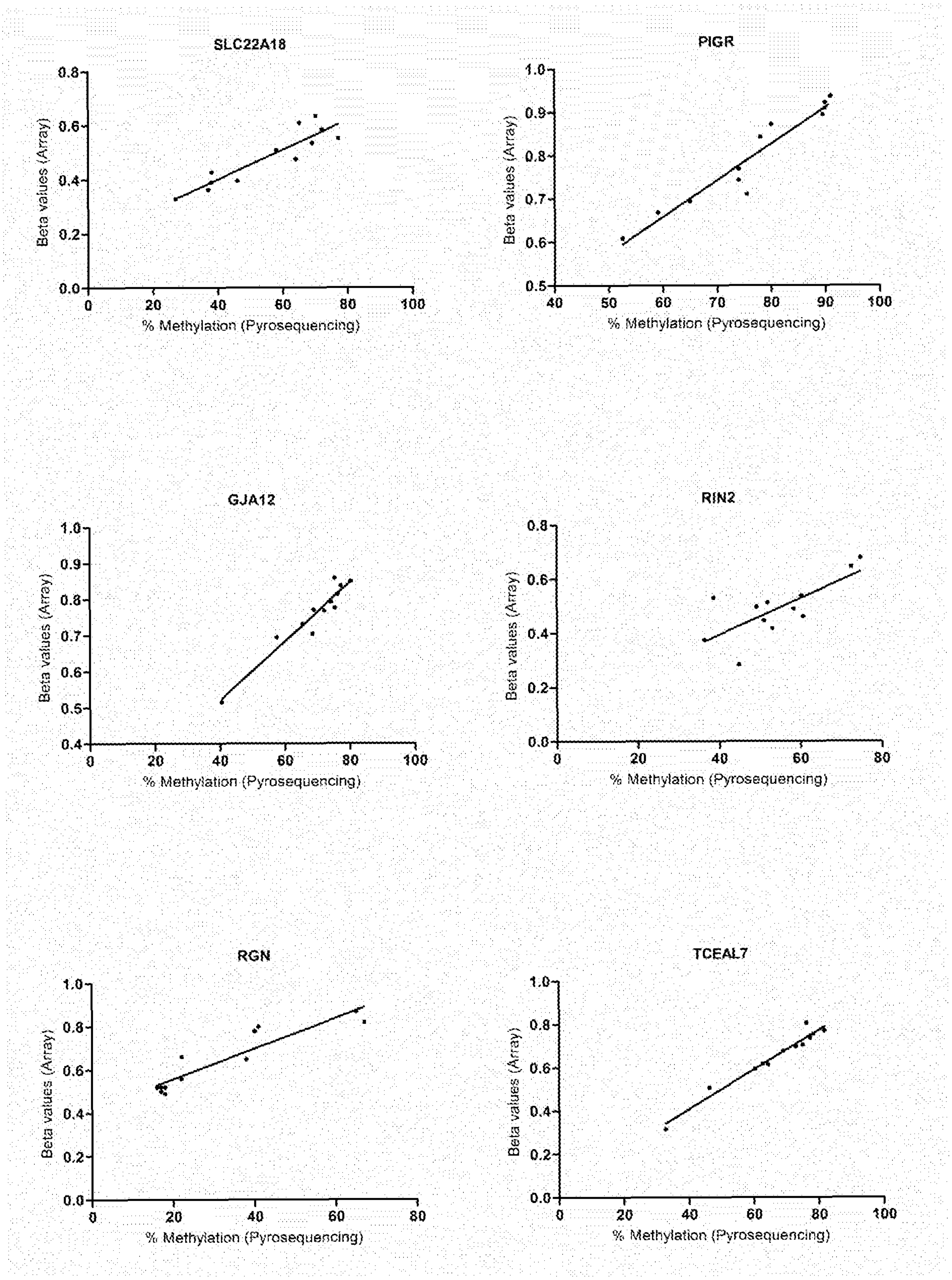
SLC22A18
Beta values (Array)
0.0 0.2 0.4 0.6 0.8
0 20 40 60 80 100
% Methylation (Pyrosequencing)
PIGR
Beta values (Array)
0.5 0.6 0.7 0.8 0.9 1.0
40 50 60 70 80 90 100
% Methylation (Pyrosequencing)
GJA12
Beta values (Array)
0.4 0.5 0.6 0.7 0.8 0.9 1.0
0 20 40 60 80 100
% Methylation (Pyrosequencing)
RIN2
Beta values (Array)
0.0 0.2 0.4 0.6 0.8
0 20 40 60 80
% Methylation (Pyrosequencing)
RGN
Beta values (Array)
0.0 0.2 0.4 0.6 0.8 1.0
0 20 40 60 80
% Methylation (Pyrosequencing)
TCEAL7
Beta values (Array)
0.0 0.2 0.4 0.6 0.8 1.0
0 20 40 60 80 100
% Methylation (Pyrosequencing)

Figure 4:

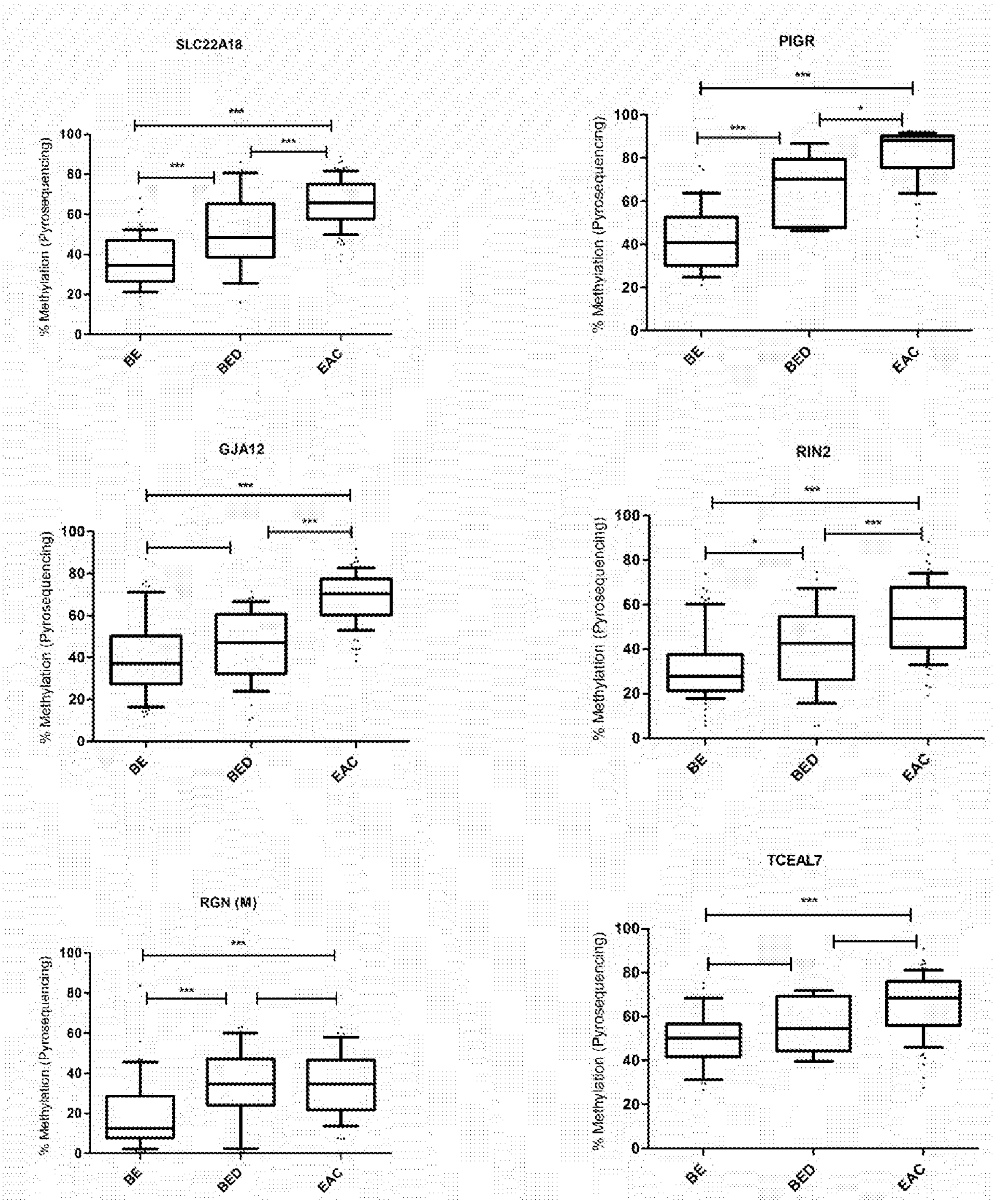
SLC22A18
PIGR
GJA12
RIN2
RGN (M)
TCEAL7
% Methylation (Pyrosequencing)
BE
BED
EAC
***
*
0
20
40
60
80
100

Figure 5:

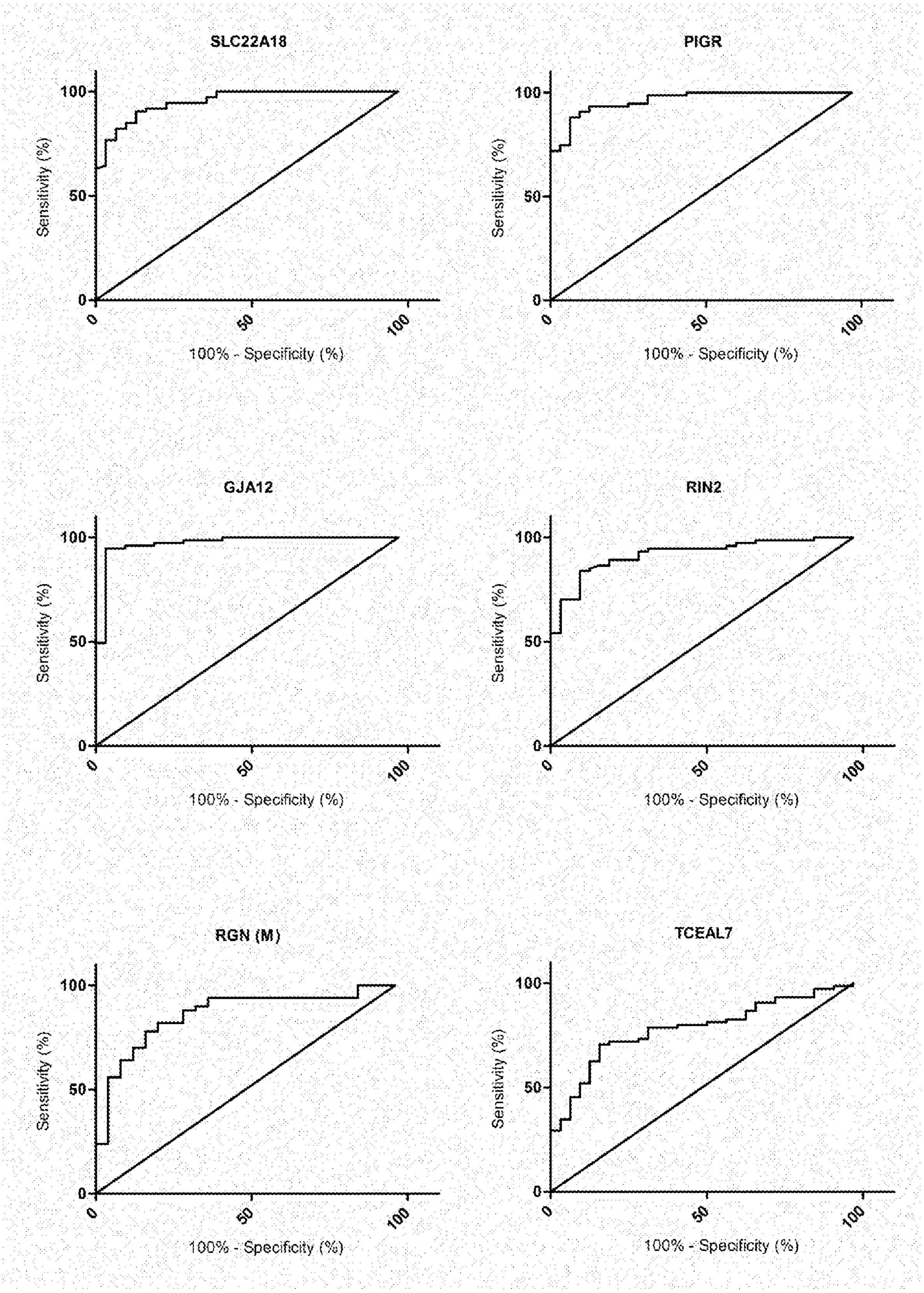
SLC22A18
PIGR
GJA12
RIN2
RGN (M)
TCEAL7
Sensitivity (%)
100% - Specificity (%)
0
50
100

Figure 6:

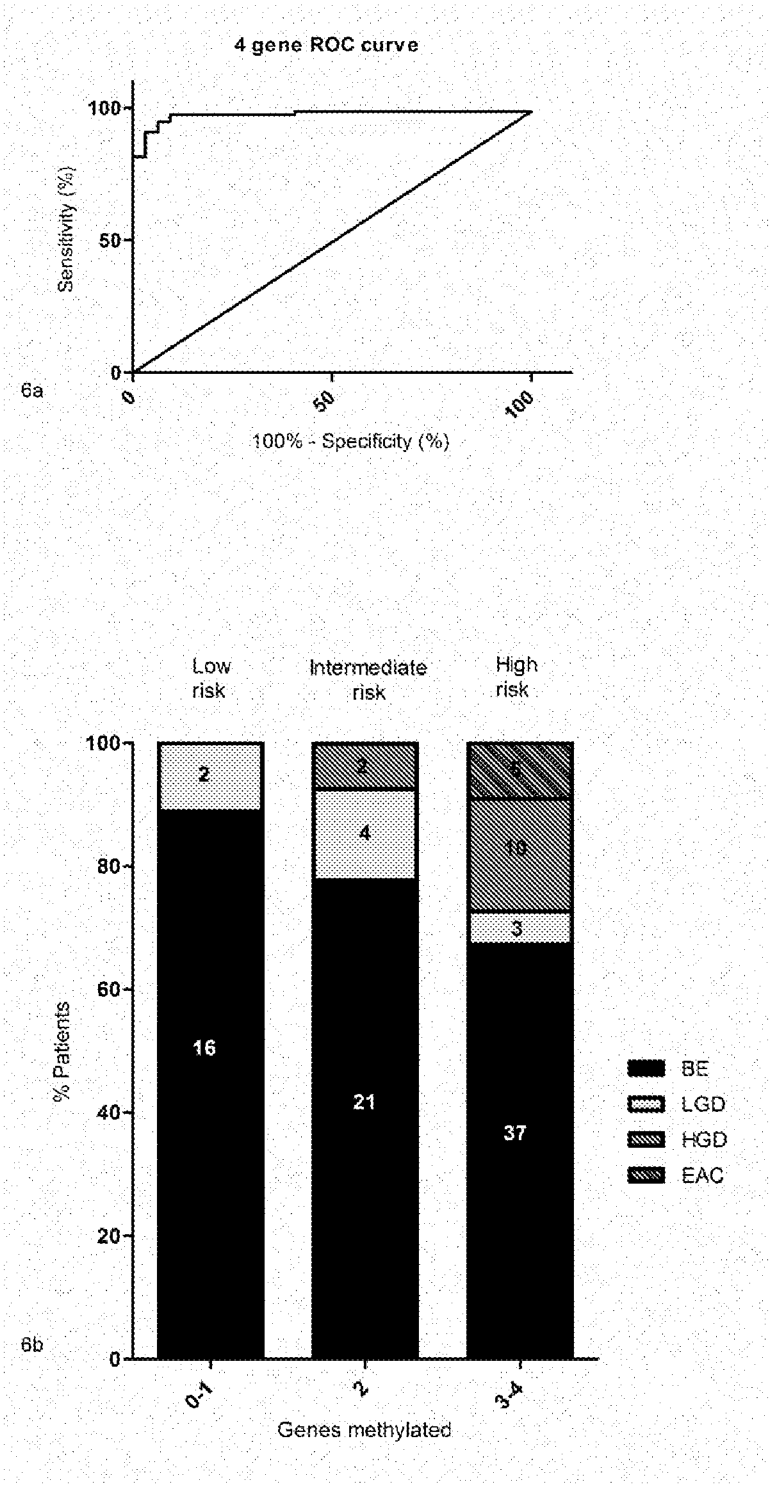
4 gene ROC curve
Sensitivity (%)
100
50
0
6a
0
50
100
100% - Specificity (%)
Low risk
Intermediate risk
High risk
100
80
60
40
20
0
% Patients
2
16
2
4
21
10
3
37
BE
LGD
HGD
EAC
6b
0-1
2
3-4
Genes methylated

Figure 7: Supplementary Figure 1:

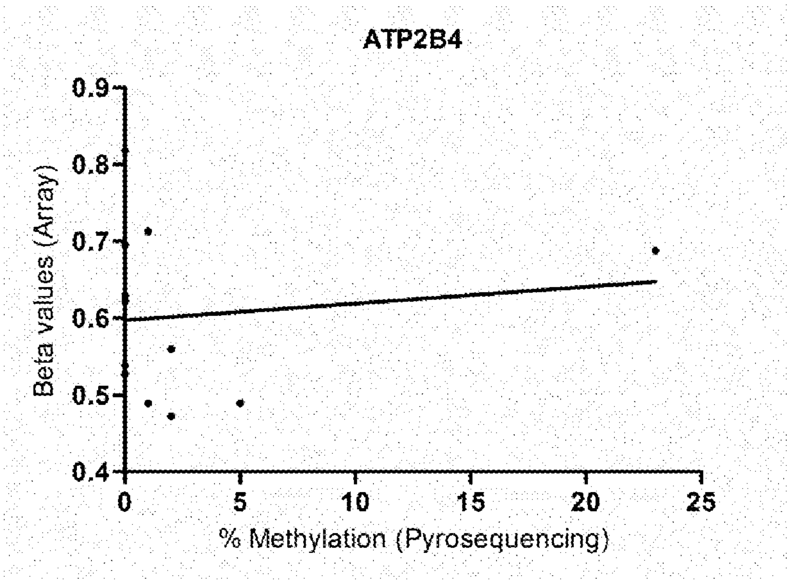


Figure 8: Supplementary Figure 2:

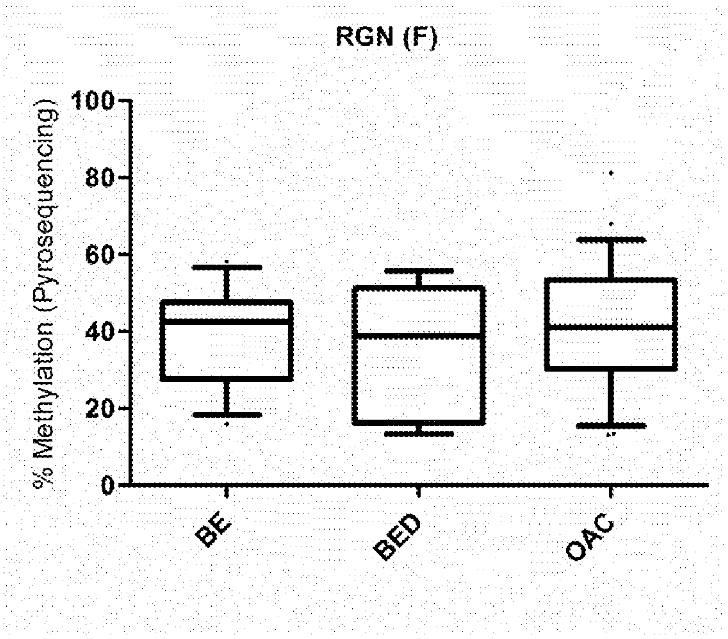

Figure 9: Supplementary Table 1:

| | | | |
|---|---|---|---|
| **BE** | **Sex** | Male | 15 |
| | | Female | 7 |
| | **Age (yrs.)** | Mean | 70 |
| | | Median | 73 |
| | | Range | 34-81 |
| **EAC** | **Sex** | Male | 20 |
| | | Female | 4 |
| | **Age (yrs.)** | Mean | 71 |
| | | Median | 75 |
| | | Range | 44-87 |
| | **Differentiation** | Well | 1 |
| | | Moderate | 9 |
| | | Poor | 13 |
| | | Unknown | 1 |
| | **Neo-adjuvant chemotherapy** | Yes | 8 |
| | | No | 15 |
| | | Unknown | 1 |

Figure 10: Supplementary Table 2:

| | | | |
|---|---|---|---|
| **BE** | **Sex** | Male | 39 |
| | | Female | 10 |
| | | Unknown | 14 |
| | **Age** | Mean | 66 |
| | | Median | 66 |
| | | Range | 30-86 |
| **BED** | **Sex** | Male | 28 |
| | | Female | 5 |
| | | Unknown | 4 |
| | **Age** | Mean | 64 |
| | | Mean | 64 |
| | | Range | 38-89 |
| **EAC** | **Sex** | Male | 68 |
| | | Female | 21 |
| | | Unknown | 4 |
| | **Chemotherapy** | Yes | 33 |
| | | No | 59 |
| | | Unknown | 2 |
| | **Age (yrs.)** | Mean | 66 |
| | | Median | 68 |
| | | Range | 44-89 |
| | **Differentiation** | Well | 3 |
| | | Moderate | 31 |
| | | Poor | 45 |
| | | Unknown | 15 |

Figure 11: Supplementary Table 3:

| | | | |
|---|---|---|---|
| **BE** | **Sex** | Male | 57 |
| | | Female | 22 |
| | **Age** | Mean | 65.8 |
| | | Median | 66 |
| | | Range | 43-86 |
| **BED/EAC** | **Sex** | Male | 19 |
| | | Female | 5 |
| | **Age** | Mean | 66.19 |
| | | Median | 64 |
| | | Range | 44-86 |

Figure 12: Supplementary Table 4:

| All genes (22BE vs. 24EAC) | | Imprinted genes (22BE vs. 24 EAC) | | X-chr genes (15BE vs. 20 EAC, males) | | X-chr genes (7BE vs. 4EAC, females) | |
|---|---|---|---|---|---|---|---|
| Signal2Noise | Wilcoxon tests | Signal2Noise | Wilcoxon tests | Signal2Noise | Wilcoxon tests | Signal2Noise | Wilcoxon tests |
| GJA12* | GJA12* | GNAS | GNAS | TCEAL7* | TCEAL7* | AFF2 | AFF2 |
| PIGR* | PIGR* | SLC22A18* | SLC22A18* | TIMP1 | RGN* | APXL | AR |
| RIN2* | GDF5 | GNAS | DLX5 | DDX3X | KLHL13 | OTEX | PCSK1N |
| FAM101A | ADORA1 | KCNQ1 | KCNQ1 | U2AF1L2 | NXF3 | NYX | ESX1 |
| HNRPUL1 | TGFB3 | CPA4 | GNAS | FUNDC1 | ATP11C | POU3F4 | RAB33A |
| COX6A1 | FAM101A | PLAGL1 | GRB10 | KLHL13 | ITM2A | AR | APXL |
| CD96 | FAM107A | GRB10 | PLAGL1 | TRAPPC2 | NDUFB11 | ZIC3 | MAGED4 |
| RANBP6 | ATP2B4* | ANKRD11 | CPA4 | TIMM8A | ESX1 | WDR40B | NYX |
| GDF5 | C7orf29 | DLX5 | ZNF264 | CRSP2 | PGK1 | SMARCA1 | PNCK |
| C7orf29 | CRIM1 | BLCAP | CDKN1C | NDUFA1 | BGN | PNCK | BEX2 |
| COX7C | TCEAL7 | NAP1L5 | RBP5 | UBE1 | CETN2 | ATG4A | NGFRAP1 |
| CCDC5 | RIN2* | UBE3A | NAP1L5 | ATP11C | STAG2 | LDOC1 | TCEAL7* |
| MYOZ3 | CDH5 | ZNF264 | NNAT | RENBP | CXorf56 | SYN1 | SLITRK4 |
| CLDN9 | AQP1 | CDKN1C | TCEB3C | SLC16A2 | ZNF185 | TCEAL7* | SLC6A8 |
| PHACS | SPARCL1 | RBP5 | WT1 | CXorf26 | APXL | SLITRK4 | CNKSR2 |
| TGFB3 | FBP2 | OSBPL5 | NDN | EFNB1 | NDUFA1 | SRPX2 | USP51 |
| SHRM | GNAS | ABCA1 | DIRAS3 | CXorf56 | PRAF2 | PCSK1N | GSPT2 |
| FAM107A | C9orf24 | NNAT | PEG3 | STAG2 | XK | ESX1 | GPR101 |
| ACVR1 | CD96 | PHLDA2 | ZIM2 | LOC203427 | MCF2 | CNKSR2 | ARMCX4 |
| ZBTB26 | ADORA2A | ZIM2 | UBE3A | ZNF75 | LOC203427 | KLHL13 | SRPX2 |
| FLAD1 | SHRM | PEG3 | ANKRD11 | HDHD1A | PRRG3 | HCFC1 | RAB39B |
| SEL1L | CP | INPP5F | KCNQ1DN | PRAF2 | KIF4A | CA5B | MAGEE1 |
| WBP1 | FES | NDN | H19 | HDAC6 | NSBP1 | BEX2 | MSN |
| FAM3C | EDG4 | TCEB3C | MEG3 | BGN | RP2 | SLC6A8 | WNK3 |
| ATP2B4* | FLJ34503 | DIRAS3 | IGF2AS | NSBP1 | SRPX | ZNF185 | ARX |
| FES | CPNE6 | WT1 | ABCA1 | HPRT1 | TSPYL2 | ATP6AP1 | SOX3 |
| AP1GBP1 | TMEM119 | HYMAI | INS | BRWD3 | RPL36A | BEX1 | SMARCA1 |
| ADORA1 | WFIKKN2 | KCNQ1DN | SNRPN | PIM2 | TCEAL3 | WNK3 | RGN* |
| HMGN2 | CLDN9 | MEG3 | OSBPL5 | CETN2 | APEX2 | RGN* | TMEM27 |
| PSMB5 | STAT5A | INS | KCNK9 | RGN* | FSHPRH1 | GLUD2 | BEX1 |

Figure 13: Supplementary Table 5:

| Assay name | CpG co-ordinates | Distance to TSS | Forward primer | Reverse primer | Sequencing primer |
|---|---|---|---|---|---|
| SLC22A18 (NM_183233.1) | 2877752 (+strand) | 225 | GTATATTTTTGGGTAGTTTGGTAAT | B-ATAAACCCTTACTCAATAAAACAAA | AGTTTTTTTTAGGGAATTTA |
| PIGR (NM_002644.2) | 2.05E+08 (- strand) | 215 | GAAATAGGAAGGTGATTTGTTAA | B-TCTATTACTTAAAAATCCTCCATC | TAAAGGTTTTGTAGTTAGAA |
| GJA12 (NM_020435.2) | 2.26E+08 (+strand) | 223 | AGGGTGTTGGGGTTTATGTATG | B-CCTAAAAAACCCTCCCTCC | GTTGTTTTAGGAGATAGTTT |
| RIN2 (NM_018993.2) | 19817644 (+strand) | 566 | GTTAATAGAAGGGTATTGGGAAGA | B-ATTACCCTACC TCTAAATTTTCCTC | TTATTGTTTATAGGGATTAG |
| RGN ( NM_004683.4) | 46822773 (+ strand) | 54 | GTTTTTTTTTTGGAAAGGAAAGGT | B-ATTAAACCTACCTCCTCCCTAAC | TTTGGAAAGGAAAGGT |
| TCEAL7 ( NM_152278.1) | 102471609 (+ strand) | 212 | GGTTAATATAATTTTGGGAAAAT | B-CCCTATCACTCATTTTTATCTTTA | GTAAATTTTGATTATAGGGA |

Figure 14: Supplementary Table 6:

| Gene | Forward primer | Reverse primer | Annealing temperature (°C) | Product size (bp) |
|---|---|---|---|---|
| SLC22A18 | CCACCACACCTCAGCGCCAG | AGCCGAGAGCCCAGGTCCAG | 64 | 313 |
| PIGR | TCCTGCCACCTGTGCCCCAT | AGGGCCACTCTCCCACAGGC | 64 | 377 |
| GJA12 | GAGCCCAGACTGCCGGAGGA | TCCCAGAGCAGACCCCACCG | 64 | 654 |
| RIN2 | GGGGTGAGGTCATCCTTAGCTC | GATGCCCTAGCAGGGGAAATTC | 65 | 288 |
| RGN | GGA GGC GTT TCC CCT CCC CT | GGG GAC GGG GGT GCC AAA TG | 65 | 517 |
| TCEAL7 | GCTGAGAGGCGATTGGATGGAGG | ACCCAAGGAGAAAGAAGCCCGGG | 64 | 396 |

Figure 15: Supplementary Table 7:

| Genes | Methylation cut-off (%) | Sensitivity (%) | Specificity (%) |
|---|---|---|---|
| GJA12 | 51.74000 | 94.700 | 96.900 |
| SLC22A18 | 49.25000 | 90.400 | 87.100 |
| PIGR | 64.755000 | 88.000 | 93.800 |
| RIN2 | 37.85500 | 83.800 | 90.600 |
| *RGN* (males only) | 18.645000 | 82.000 | 80.000 |
| TCEAL7 | 58.54000 | 70.700 | 84.400 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 20091005533 A, UNIV JOHNS HOPKING
- US 2012094287 A, MARKOWITZ SANFORD D
- WO 2007045896 A
- WO 2011058316 A


### Non-patent literature cited in the description


- **JIN et al.** *Cancer Research,* 2009, vol. 69, 4112-4115
- **KAZ et al.** *Epigenetics,* 2011, vol. 6, 1403-1412
- **ALVI et al.** Gastroenterology. Digestive Disease Week (DDW), 2012, vol. 142, S443
- **KAZ et al.** *EPIGENETICS LANDES BIOSCIENCE USA,* 2011, vol. 6 (12), ISSN 1559-2294, 1403-1412
- **JIN et al.** GASTROENTEROLOGY. ELSEVIER, 2009, vol. 136, A - 36
- **EADS, C.A. et al.** MethyLight: a high-throughput assay to measure DNA methylation. *Nucleic Acids Res,* 2000, vol. 28 (E32
- **HVID-JENSEN F ; PEDERSEN L ; DREWES AM ; SØRENSEN HT ; FUNCH-JENSEN P.** Incidence of Adenocarcinoma among Patients with Barrett's Esophagus. *New England Journal of Medicine,* 2011, vol. 365, 1375-1383
- **POHL H ; SIROVICH B ; WELCH HG.** Esophageal Adenocarcinoma Incidence: Are We Reaching the Peak?. *Cancer Epidemiology Biomarkers & Prevention,* 2010, vol. 19, 1468-1470
- **CRANE SJ ; LOCKE GR ; HARMSEN WS ; ZINSMEISTER AR ; ROMERO Y ; TALLEY NJ.** Survival Trends in Patients With Gastric and Esophageal Adenocarcinomas: A Population-Based Study. *Mayo Clinic Proceedings,* 2008, vol. 83, 1087-1094
- **SPECHLER SJ ; SHARMA P ; SOUZA RF ; INADOMI JM ; SHAHEEN NJ.** American Gastroenterological Association Technical Review on the Management of Barrett's Esophagus. *Gastroenterology,* 2011, vol. 140, e18-e52
- **DEMEESTER S.** Evaluation and Treatment of Superficial Esophageal Cancer. *Journal of Gastrointestinal Surgery,* 2010, vol. 14, 94-100
- **YOUSEF F ; CARDWELL C ; CANTWELL MM ; GALWAY K ; JOHNSTON BT ; MURRAY L.** The Incidence of Esophageal Cancer and High-Grade Dysplasia in Barrett's Esophagus: A Systematic Review and Meta-Analysis. *American Journal of Epidemiology,* 2008, vol. 168, 237-249
- **KAHRILAS PJ.** The Problems with Surveillance of Barrett's Esophagus. *New England Journal of Medicine,* 2011, vol. 365, 1437-1438
- **DESAI TK ; KRISHNAN K ; SAMALA N ; SINGH J ; CLULEY J ; PERLA S ; HOWDEN CW.** The incidence of oesophageal adenocarcinoma in non-dysplastic Barrett's oesophagus: a meta-analysis. *Gut,* 2011
- **GOLDBLUM JR.** Controversies in the Diagnosis of Barrett Esophagus and Barrett-Related Dysplasia: One Pathologist's Perspective. *Archives of Pathology & Laboratory Medicine,* 2010, vol. 134, 1479-1484
- **DOWNS-KELLY E ; MENDELIN JE ; BENNETT AE ; CASTILLA E ; HENRICKS WH ; SCHOENFIELD L ; SKACEL M ; YERIAN L ; RICE TW ; RYBICKI LA.** Poor Interobserver Agreement in the Distinction of High-Grade Dysplasia and Adenocarcinoma in Pretreatment Barrett's Esophagus Biopsies. *Am J Gastroenterol,* 2008, vol. 103, 2333-2340
- **CURVERS WL ; TEN KATE FJ ; KRISHNADATH KK ; VISSER M ; ELZER B ; BAAK LC ; BOHMER C ; MALLANT-HENT RC ; VAN OIJEN A ; NABER AH.** Low-grade dysplasia in Barrett's esophagus: overdiagnosed and underestimated. *Am J Gastroenterol,* 2010, vol. 105, 1523-30
- **WANI S ; FALK GW ; POST J ; YERIAN L ; HALL M ; WANG A ; GUPTA N ; GADDAM S ; SINGH M ; SINGH V.** Risk Factors for Progression of Low-Grade Dysplasia in Patients With Barrett's Esophagus. *Gastroenterology,* 2011, vol. 141, 1179-1186

- **SHAHEEN NJ ; SHARMA P ; OVERHOLT BF ; WOLFSEN HC ; SAMPLINER RE ; WANG KK ; GALANKO JA ; BRONNER MP ; GOLDBLUM JR ; BENNETT AE.** Radiofrequency Ablation in Barrett's Esophagus with Dysplasia. *New England Journal of Medicine,* 2009, vol. 360, 2277-2288
- **ESTELLER M.** Epigenetics in Cancer. *New England Journal of Medicine,* 2008, vol. 358, 1148-1159
- **SCHULMANN K ; STERIAN A ; BERKI A ; YIN J ; SATO F ; XU Y ; OLARU A ; WANG S ; MORI Y ; DEACU E.** Inactivation of p16, RUNX3, and HPP1 occurs early in Barrett's-associated neoplastic progression and predicts progression risk. *Oncogene,* 2005, vol. 24, 4138-4148
- **CLEMENT G ; BRAUNSCHWEIG R ; PASQUIER N ; BOSMAN FT ; BENHATTAR J.** Alterations of the Wnt signaling pathway during the neoplastic progression of Barrett's esophagus. *Oncogene,* 2006, vol. 25, 3084-3092
- **JIN Z ; CHENG Y ; OLARU A ; KAN T ; YANG J ; PAUN B ; ITO T ; HAMILTON JP ; DAVID S ; AGARWAL R.** Promoter hypermethylation of CDH13 is a common, early event in human esophageal adenocarcinogenesis and correlates with clinical risk factors. *International Journal of Cancer,* 2008, vol. 123, 2331-2336
- **HUANG Y ; PETERS CJ ; FITZGERALD RC ; GJERSET RA.** Progressive silencing of p14ARF in oesophageal adenocarcinoma. *Journal of Cellular and Molecular Medicine,* 2009, vol. 13, 398-409
- **KUESTER D ; EL-RIFAI WE ; PENG D ; RUEMMELE P ; KROECKEL I ; PETERS B ; MOSKALUK CA ; STOLTE M ; MÖNKEMÜLLER K ; MEYER F.** Silencing of MGMT expression by promoter hypermethylation in the metaplasia-dysplasia-carcinoma sequence of Barrett's esophagus. *Cancer Letters,* 2009, vol. 275, 117-126
- **JIN Z ; CHENG Y ; GU W ; ZHENG Y ; SATO F ; MORI Y ; OLARU AV ; PAUN BC ; YANG J ; KAN T.** A Multicenter, Double-Blinded Validation Study of Methylation Biomarkers for Progression Prediction in Barrett's Esophagus. *Cancer Research,* 2009, vol. 69, 4112-4115
- **TABY R ; ISSA J-PJ.** CA: A Cancer Journal for Clinicians. *Cancer Epigenetics,* 2010, vol. 60, 376-392
- **LAIRD PW.** Principles and challenges of genome-wide DNA methylation analysis. *Nat Rev Genet,* 2010, vol. 11, 191-203
- Controlling the False Discovery Rate: A Practical and Powerful Approach to Multiple Testing. **BENJAMINI Y ; HOCHBERG Y.** Methodological. Journal of the Royal Statistical Society, 1995, vol. 57, 289-300
- **WANG JS ; GUO M ; MONTGOMERY EA ; THOMPSON RE ; COSBY H ; HICKS L ; WANG S ; HERMAN JG ; CANTO MI.** DNA Promoter Hypermethylation of p16 and APC Predicts Neoplastic Progression in Barrett's Esophagus. *Am J Gastroenterol,* 2009, vol. 104, 2153-2160
- **JIN Z ; CHENG Y ; GU W ; ZHENG Y ; SATO F ; MORI Y ; OLARU AV ; PAUN BC ; YANG J ; KAN T.** A Multicenter, Double-Blinded Validation Study of Methylation Biomarkers for Progression Prediction in Barrett's Esophagus. *Cancer Res,* 2009, vol. 69, 4112-4115
- **JIN Z ; HAMILTON JP ; YANG J ; MORI Y ; OLARU A ; SATO F ; ITO T ; KAN T ; CHENG Y ; PAUN B.** Hypermethylation of the AKAP12 Promoter is a Biomarker of Barrett's-Associated Esophageal Neoplastic Progression. *Cancer Epidemiol Biomarkers Prev,* 2008, vol. 17, 111-117
- **JIN Z ; OLARU A ; YANG J ; SATO F ; CHENG Y ; KAN T ; MORI Y ; MANTZUR C ; PAUN B ; HAMILTON JP.** Hypermethylation of Tachykinin-1 Is a Potential Biomarker in Human Esophageal Cancer. *Clin Cancer Res,* 2007, vol. 13, 6293-6300
- **TOST J ; GUT IG.** DNA methylation analysis by pyrosequencing. *Nat. Protocols,* 2007, vol. 2, 2265-2275
- **ALVAREZ H ; OPALINSKA J ; ZHOU L ; SOHAL D ; FAZZARI MJ ; YU Y ; MONTAGNA C ; MONTGOMERY EA ; CANTO M ; DUNBAR KB.** Widespread Hypomethylation Occurs Early and Synergizes with Gene Amplification during Esophageal Carcinogenesis. *PLoS Genet,* 2011, vol. 7, e1001356
- **CHU S-H ; FENG D-F ; MA Y-B ; ZHANG H ; ZHU Z-A ; LI Z-Q ; JIANG P-C.** Promoter methylation and downregulation of SLC22A18 are associated with the development and progression of human glioma. *Journal of Translational Medicine,* 2011, vol. 9, 156
- **GALLAGHER E ; MC GOLDRICK A ; CHUNG WY ; MC CORMACK O ; HARRISON M ; KERIN M ; DERVAN PA ; MC CANN A.** Gain of imprinting of SLC22A18 sense and antisense transcripts in human breast cancer. *Genomics,* 2006, vol. 88, 12-17
- **SCHWIENBACHER C ; GRAMANTIERI L ; SCELFO R ; VERONESE A ; CALIN GA ; BOLONDI L ; CROCE CM ; BARBANTI-BRODANO G ; NEGRINI M.** Gain of imprinting at chromosome 11p15: A pathogenetic mechanism identified in human hepatocarcinomas. *Proceedings of the National Academy of Sciences,* 2000, vol. 97, 5445-5449
- **YAMAGUCHI M ; DAIMON Y.** Overexpression of regucalcin suppresses cell proliferation in cloned rat hepatoma H4-II-E cells: Involvement of intracellular signaling factors and cell cycle-related genes. *Journal of Cellular Biochemistry,* 2005, vol. 95, 1169-1177

- **MAIA C ; SANTOS C ; SCHMITT F ; SOCORRO S.** Regucalcin is under-expressed in human breast and prostate cancers: Effect of sex steroid hormones. *Journal of Cellular Biochemistry,* 2009, vol. 107, 667-676
- **KERKHOF M ; VAN DEKKEN H ; STEYERBERG EW ; MEIJER GA ; MULDER AH ; DE BRUÏNE A ; DRIESSEN A ; TEN KATE FJ ; KUSTERS JG ; KUIPERS EJ.** Grading of dysplasia in Barrett's oesophagus: substantial interobserver variation between general and gastrointestinal pathologists. *Histopathology,* 2007, vol. 50, 920-927
- **BELSHAW NJ ; ELLIOTT GO ; FOXALL RJ ; DAINTY JR ; PAL N ; COUPE A ; GARG D ; BRADBURN DM ; MATHERS JC ; JOHNSON IT.** Profiling CpG island field methylation in both morphologically normal and neoplastic human colonic mucosa. *Br J Cancer,* 2008, vol. 99, 136-142
- **SHEN L ; KONDO Y ; ROSNER GL ; XIAO L ; HERNANDEZ NS ; VILAYTHONG J ; HOULIHAN PS ; KROUSE RS ; PRASAD AR ; EINSPAHR JG.** MGMT Promoter Methylation and Field Defect in Sporadic Colorectal Cancer. *Journal of the National Cancer Institute,* 2005, vol. 97, 1330-1338
- **LEEDHAM SJ ; PRESTON SL ; MCDONALD SAC ; ELIA G ; BHANDARI P ; POLLER D ; HARRISON R ; NOVELLI MR ; JANKOWSKI JA ; WRIGHT NA.** Individual crypt genetic heterogeneity and the origin of metaplastic glandular epithelium in human Barrett's oesophagus. *Gut,* 2008, vol. 57, 1041-1048
- **MERLO LMF ; SHAH NA ; LI X ; BLOUNT PL ; VAUGHAN TL ; REID BJ ; MALEY CC.** A Comprehensive Survey of Clonal Diversity Measures in Barrett's Esophagus as Biomarkers of Progression to Esophageal Adenocarcinoma. *Cancer Prevention Research,* 2010, vol. 3, 1388-1397
- **REID BJ ; KOSTADINOV R ; MALEY CC.** New Strategies in Barrett's Esophagus: Integrating Clonal Evolutionary Theory with Clinical Management. *Clinical Cancer Research,* 2011, vol. 17, 3512-3519